(19) Europäisches Patentamt
European Patent Office
Office européen des brevets

(11) **EP 4 458 337 B1**

(12) **EUROPEAN PATENT SPECIFICATION**

(45) Date of publication and mention
of the grant of the patent:
**09.04.2025 Bulletin 2025/15**

(21) Application number: **24172809.6**

(22) Date of filing: **26.04.2024**

(51) International Patent Classification (IPC):
$A61F\ 13/494^{(2006.01)}$ $A61F\ 13/496^{(2006.01)}$
$A61F\ 13/505^{(2006.01)}$

(52) Cooperative Patent Classification (CPC):
**A61F 13/49446; A61F 13/49466**

(54) **ABSORBENT ARTICLE WITH ANTI-LEAKAGE TRANSVERSAL BARRIER**

ABSORBIERENDER ARTIKEL MIT QUERSPERREN GEGEN LECKS

ARTICLE ABSORBANT À BARRIÈRE TRANSVERSALE ANTI-FUITE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: 27.04.2023 EP 23170461
10.07.2023 PCT/EP2023/069091
15.01.2024 EP 24151960
21.02.2024 EP 24158980
21.03.2024 EP 24165293
27.03.2024 EP 24167029

(43) Date of publication of application:
**06.11.2024 Bulletin 2024/45**

(73) Proprietors:
• **Ontex BV**
**9255 Buggenhout (BE)**

• **Ontex Group NV**
**9320 Erembodegem (BE)**

(72) Inventors:
• **MOHAMMED, Moula Saheb**
**56727 Mayen (DE)**
• **SCHULTE, Lea**
**56727 Mayen (DE)**
• **PSCHYKLENK, Lukas**
**56761 Müllenbach (DE)**

(74) Representative: **Macchetta, Andrea**
**Ontex BV**
**Korte Keppestraat 21**
**9320 Erembodegem-Aalst (BE)**

(56) References cited:
**US-A1- 2018 338 870     US-B2- 7 204 830**

**Description**

**TECHNICAL FIELD**

The present disclosure is directed to an absorbent article for personal hygiene, typically in the form of pants (such as for babies, youths or adults) and generally of the disposable kind as well as the method for the manufacture of such absorbent articles and the apparatus to carry out such method.

**BACKGROUND**

Absorbent articles for personal hygiene are designed to absorb and contain bodily exudates, such as a large quantity of urine and/or stool. Non-limiting examples of disposable absorbent articles include diapers, pants, training pants, pad, adult incontinence products, and feminine hygiene products (including, for example, sanitary napkins and tampons). Other examples of disposable absorbent articles include bandages and wound dressings. In some embodiments, for example, an absorbent article comprises several layers providing different functions, for example a topsheet, a backsheet and in-between an absorbent core, among other layers.

The function of the absorbent core is to absorb and retain the exudates for a prolonged amount of time, for example overnight for a diaper, minimize re-wet to keep the wearer dry and avoid soiling of clothes or bed sheets. The majority of currently marketed absorbent articles comprise as absorbent material a blend of comminuted wood pulp (also referred to as fluff pulp and/or cellulose fibers) with superabsorbent polymers (SAP) in particulate form, also called absorbent gelling materials (AGM), see for example U.S. Pat. No. 5,151,092 (Buell). Absorbent articles having a core consisting essentially of SAP as absorbent material (so called "airfelt-free" cores) have also been proposed but are less common than traditional mixed cores (see e.g. WO2008/155699 and WO2012/052172).

A number of disclosures exist (see for example EP3451989 B1) directed to limiting the risk of leakage by providing transversal barriers at the front and/or back of a diaper. However whilst such executions may be effective on diaper constructs they are less effective and or complex/costly to incorporate into a pant concept.

An attempt to overcome such shortcomings has been made in for example WO2021/170027 A1 wherein a nonwoven layer of the front and/or back belt is folded a plurality of times to form a waist guard. Although this provides for an effective way to introduce a transversal barrier in a pant construct, there are several drawbacks that have been identified: firstly, the nonwoven that is generally hydrophobic may be sufficient to capture solids but is less effective to capture larger amounts of liquid exudates (particularly under pressure such as when a baby is moving and/or application of sudden pressure such as sitting/dropping on the floor on its buttocks); secondly it limits the positioning thereof to a closer position at the waist edge which is less effective in providing a barrier since it is positioned further away from the core and to position it closer to the core and away from the waist edge would require a significant increase in length of the nonwoven which adds significant waste and cost; thirdly applying the multiple folds at different positions of the same component and especially further elasticizing the region when/if desired may add significant process complexity particularly at high speeds.

A need therefore exists for improved absorbent articles having leakage protection particularly for substantially liquid exudates such as urine and/or liquid stool yet in in a cost-effective manner and with more limited additional waste as well as having an efficient method and apparatus for the manufacture of such absorbent articles.

**SUMMARY**

In a first aspect the disclosure relates to an absorbent article for personal hygiene, preferably a disposable pant, comprising: a substantially transversely extending front panel; a substantially transversely extending back panel; and a substantially longitudinally extending absorbent insert joined to each of said front and back panels, said absorbent insert comprising front and back transversal edges and left and right longitudinal edges connecting the front and back transversal edges to form a perimeter thereof; and wherein said insert comprises an absorbent core; wherein the front and back panels are joined together to define a pair of side seams, and said article comprises a transversal waist barrier proximal to the front and/or back transversal edges of the absorbent insert wherein said barrier comprises a plurality of liquid retarding layers and wherein said plurality of liquid retarding layers comprise at least two layers selected from the group consisting of hydrophobic nonwovens, films, and/or strands, and at least a majority of the waist barrier comprises at least two of said plurality of liquid retarding layers and wherein said at least two of said plurality of liquid retarding layers are un-joined at one or more locations so that said layers are separated from one another at said location(s) by a gap, and preferably wherein said one or more locations together correspond to a majority of a total surface of said waist barrier; the article preferably having a Seepage Index of less than 35% according to the Seepage Index Method herein.

According to an embodiment, the absorbent article, preferably a disposable pant, comprising: a front panel preferably substantially transversely extending; a back panel preferably substantially transversely extending; and an absorbent insert, preferably substantially longitudinally extending, joined to each of said front and back panels, said absorbent insert comprising front and back transversal edges and left and right longitudinal edges connecting the front and back transversal edges to form a perimeter thereof and generally wherein the left and right longitudinal edges extend substantially along the longitudinal axis y and are oppositely disposed such that said longitudinal axis y extends therebetween, and the front and back transversal edges extend substantially along the transversal axis x, and typically substantially perpendicular to the longitudinal axis y, and are oppositely disposed such that said transverse axis x extends therebetween). The front and back panels are joined together to define a pair of side seams, and at least a portion of said absorbent insert proximal to the front and/or back transversal edges thereof comprises a transversal waist barrier comprising a substantially liquid impermeable film layer.

According to an embodiment, the plurality of liquid retarding layers comprise at least two, preferably more than two, layers selected from the group consisting of hydrophobic nonwovens, films, and/or strands; more preferably a laminate comprising a film or a plurality of strands, preferably the film or strands being elastic, sandwiched between two of said hydrophobic nonwovens.

A single nonwoven may be folded to provide a plurality of nonwoven layers or distinct nonwoven layers that are directly or indirectly joined together may be used.

The nonwoven may be an elastic nonwoven or may be a substantially non-elastic nonwoven comprising elastic adhesive.

By a "plurality of liquid retarding layers" it is typically meant herein that the such layers are discrete layers that may be joined together by a suitable bonding (or joining means) such as adhesive and/or mechanical bonding and hence a single layer of nonwoven comprising a plurality of intimately co-formed layers of fibers such as by multi-denier spinning (e.g. a laminates like spunbond-meltblown nonwovens, spunbond-meltblown-spunbond nonwovens, spunbond-meltblown-meltblown-spunbond nonwovens, and the like) are generally not encompassed within such meaning.

According to an embodiment, each hydrophobic nonwoven is a multi-layer nonwoven comprising at least two, preferably at least three, layers selected from a spunbond nonwoven and a meltblown nonwoven, more preferably said nonwoven being selected from a spunbond-meltblown nonwoven, a spunbond-meltblown-spunbond nonwoven, and a spunbond-meltblown-meltblown-spunbond nonwoven.

According to an embodiment, the hydrophobic nonwovens comprise, preferably consist of, synthetic fibers; preferably wherein said fibers comprise a material selected from polypropylene or derivatives thereof, polyethylene or derivatives thereof, polylactic acid or derivatives thereof, polylactic-co-glycolic acid or derivatives thereof, polyhydroxyalkanoates or derivatives thereof, and mixtures thereof.

At least two of the plurality of liquid retarding layers may be spaced apart, typically such to form a gap therebetween. The gap may be free of adhesive and/or mechanical bonds and such that said layers are not joined together at said gap preferably to form an air cushion or air channel. Advantageously the presence of such gap may add a further barrier to liquid leakage thanks to the absence of contact between layers that retards liquid from one layer to reach the next.

According to an embodiment, the opening is positioned closer to a transversal centerline (x) than the joined portions of the perimeter of the patch.

According to an embodiment, said barrier (5) is breathable and has a water vapor transmission rate (WVTR) of less than about 2300 $g/m^2x24hr$ at an elongation of about 0% and a water vapor transmission rate (WVTR) of more than about 2800 $g/m^2x24hr$ at an elongation of about 100%, according to the test method herein; preferably a water vapor transmission rate (WVTR) of less than about 2250 $g/m^2x24hr$, preferably less than about 2150 $g/m^2x24hr$, even more preferably from about 500 $g/m^2x24hr$ to about 2000 $g/m^2x24hr$, at an elongation of about 0%; and a water vapor transmission rate (WVTR) of more than about 2850 $g/m^2x24hr$, preferably more than about 2950 $g/m^2x24hr$, more preferably more than about 3000 $g/m^2x24hr$, even more preferably from about 3100 $g/m^2x24hr$ to about 5500 $g/m^2x24hr$, at an elongation of about 100%, as measured according to the test method herein.

"Elastic," "elastomeric," and "elasticized/elasticised" herein may mean the ability of a material to stretch preferably by at least 100% without rupture or breakage at a given load, and upon release of the load the elastic material or component exhibits at least 70% recovery (i.e., has less than 30% set) in one of the directions as per the Hysteresis Test described herein. Stretch, sometimes referred to as strain, percent strain, engineering strain, draw ratio, or elongation, along with recovery and set may each be determined according to the Hysteresis Test described in more detail below. Materials that are not elastic are referred as inelastic. "Extensible" typically means the ability to stretch or elongate, without rupture or breakage, by at least 50% as per step 5(a) in the Hysteresis Test herein (replacing the specified 100% strain with 50% strain). The term "extensible" refers to the property of a material, wherein: when a biasing force is applied to the material, the material can be extended to an elongated length of preferably at least 110% of its original relaxed length (i.e., can extend 10%), without a rupture or breakage that renders the material unusable for its intended purpose. A material that does not meet this definition is considered inextensible. In some embodiments, an extensible material may be able to be extended to an elongated length of 125% or more of its original relaxed length without rupture or breakage that renders the material unusable for its intended purpose. An extensible material may or may not exhibit recovery after application of a biasing force. Throughout the present disclosure, an extensible material is considered to be "elastically extensible" if, when a biasing force is applied to the material, the material can be extended to an elongated length of at least 110% of its original relaxed length (i.e., can extend 10%), without rupture or breakage which renders the material unusable for its intended purpose, and when the force is removed from the material, the material recovers at least 40% of its elongation. In various examples, when the force is removed from an elastically extensible material, the material may recover at least 60%, or at least 80%, of its elongation.

According to an embodiment, the joined portion of the perimeter of the patch is joined by adhesive and/or mechanical bonding having a shape and/or pattern that is substantially c-shaped, preferably such that at least one of the longest sides and at least two opposite portions of the shortest sides of said patch are joined and wherein at least one of the longest sides of said patch is un-joined.

According to an embodiment, said barrier comprises one or more of an elastic film and an elastic strand, and wherein the elastic film and/or strand(s) form an elastic area and wherein the elastic area is less than a patch area such that a substantially inelastic area is positioned outboard of the elastic area, preferably wherein the inelastic area comprises at least one of: an area free of elastic film and/or strand(s); and an area wherein the elastic film and/or strand(s) has been de-activated.

The barrier may comprise a combination of an impermeable film typically as described herein and that is generally substantially non-elastic; and one or more elastic strands joined to said film to render it elastic (for example by joining elastics, being strand-coated with adhesive, directly to the film). Preferably, the film comprises bio-PE (generally understood herein as bio-derived polyethylene, such generally having ethylene as monomer that is produced by fermentation of agricultural feedstocks such as sugar cane or corn). Advantageously this allows to increase the natural base content of the product without impacting negatively the stretch performance that is inherently hindered by the use of such materials in the film construction. The construction of the barrier itself for other aspects may remain as described in the embodiments herein.

According to an embodiment, at least a portion of the substantially inelastic area is joined to the absorbent insert and/or panel(s). Advantageously this may provide for optimal anchoring.

According to an embodiment, the un-joined portion of said perimeter comprises a further elastic material, preferably in the form of one or more elastic strands. Preferably, the further elastic material is positioned on a body-facing or garment facing surface of the patch so as to promote formation of an opening upon extension thereof.

The elastic area may comprise a plurality of wrinkles; and/or wherein the elastic area comprises a plurality of apertures. Advantageously the wrinkles may provide for added perceived softness and/or the formation of breathability channels therebetween, the apertures may provided enhanced breathability when in contact with the wearer's skin.

In a second aspect the disclosure relates to a method for the manufacture of an absorbent article according to any of the preceding claims, said method comprising the steps of: providing a front panel; providing a back panel; providing an absorbent insert comprising front and back transversal edges and left and right longitudinal edges connecting the front and back transversal edges to form a perimeter thereof; providing a patch or discrete layer to cover at least a region of the absorbent insert proximal to the front and/or back transversal edges to form transversal waist barrier(s) ; and either joining said absorbent insert to said front and back panels and joining said patch or discrete layer to said front and/or back panels by bonding means to cover at least a region of the absorbent insert proximal to the front and/or back transversal edges; or joining patch or discrete layer to said insert by bonding means to cover at least a region of the absorbent insert proximal to the front and/or back transversal edges and joining said insert to said front and back panels; or joining said absorbent insert to said front and back panels and joining patch or discrete layer to absorbent insert by bonding means to cover at least a region of the absorbent insert proximal to the front and/or back transversal edges; and joining said front and back panels together along a pair of oppositely disposed side seams.

According to an embodiment, the bonding means comprise applying a pattern of adhesive onto a patch or discrete layer and/or applying a pattern of adhesive onto a portion of the absorbent insert proximal to the front and/or back transversal edge said pattern of adhesive extending all along the transversal edges and partially along the longitudinal edges of the patch or discrete layer and/or absorbent insert and applying said patch or discrete layer onto the top surface of the absorbent insert at a portion of the absorbent insert being proximal to the front and/or back transversal edge to form transversal waist barrier(s) or the bonding means comprise mechanical bonding preferably ultrasonic bonding.

According to an embodiment, the total surface area of the patch or discrete layer comprising liquid impermeable film is greater than the surface area of the bonding means.

According to an embodiment, the patch or discrete layer comprises a liquid impermeable film, preferably elastic.

According to an embodiment, the patch or discrete layer comprises a laminate comprising a plurality of elastic strands sandwiched between two layers preferably two nonwoven layers.

According to an embodiment, the film and/or elastic strands form an elastic area where the total area of patch or discrete layer is greater than the elastic area, meaning the total area where the film and/or sum of elastic strands are arranged, said total area of patch or discrete layer preferably comprising an inelastic area outboard of the elastic area and substantially encircling or enclosing it, e.g. the inelastic area substantially follows the perimeter of the patch or discrete layer for example the inelastic are could be a void free of elastic film and/or strands, or comprise de-activated elastic material such as elastic material rendered inelastic by technic such as cutting or melting. Such arrangement enables to ensure a better attachment or anchoring of the elastic film and/or strands since joining would occur in areas free of additional ondulations or wrinkles.

According to an embodiment, the film is mechanically bonded, preferably by ultrasonic bonding, such to form openings at the bonding sites/regions.

According to an embodiment, the film is mechanically bonded, preferably by ultrasonic bonding, such to form openings at the bonding sites or regions, such bonding ensuring greater breathability.

In an aspect, the disclosure relates to a method for the manufacture of an absorbent article as described hereabove, said method comprising the, preferably sequential, steps of:

- providing a front panel comprising an outer nonwoven and an inner nonwoven, for example a front outer nonwoven and a front inner nonwoven
- providing a back panel comprising a back outer nonwoven and a back inner nonwoven, for example a back outer nonwoven and a back inner nonwoven;
- providing an absorbent insert comprising front and back transversal edges and left and right longitudinal edges connecting the front and back transversal edges to form a perimeter thereof;
- joining said absorbent insert to said front and back panels, namely to the inner nonwovens, namely to the front and back inner nonwoven,
- folding both outer nonwoven and inner nonwoven of the front panel over a portion of the front transversal edge of the absorbent insert and/or folding both outer nonwoven and inner nonwoven of the back panel over a portion of the back transversal edge of the absorbent insert, namely folding front outer nonwoven and front inner nonwoven over a portion of the front transversal edge of the absorbent insert and/or folding back outer nonwoven and back inner nonwoven over a portion of the back transversal edge of the absorbent insert,
- joining said front and back panels together along a pair of oppositely disposed side seams.

In an embodiment, the disclosure relates to a method for the manufacture of an absorbent article comprising the steps of: providing a front panel; providing a back panel; providing an absorbent insert comprising front and back transversal edges and left and right longitudinal edges connecting the front and back transversal edges to form a perimeter thereof and generally wherein the left and right longitudinal edges extend substantially along the longitudinal axis y and are oppositely disposed such that said longitudinal axis y extends therebetween, and the front and back transversal edges extend substantially along the transversal axis x, and typically substantially perpendicular to the longitudinal axis y, and are oppositely disposed such that said transverse axis x extends therebetween); folding a portion of said absorbent insert proximal to the front and/or back transversal edges thereof onto itself, preferably in the shape of a substantially U-fold, to form transversal waist barrier(s); joining said insert to said front and back panels; and joining said front and back panels together along a pair of oppositely disposed side seams. Preferably, said folding step is carried out by static folding where a static element, *i.e.* a stator, such as a stationary folding blade deflects a portion of said absorbent insert proximal to the front and/or back transversal edges thereof onto itself. In other words, the folding is step is preferably done by passive folding meaning that the folding is done with a moving element, i.e. the absorbent insert, being folded by an unmoving, or immobile or fixed element, e.g. a blade. Such folding is more cost efficient and more reproducible. The present disclosure encompasses embodiments where the folding step is carried out by dynamic folding, where a mobile element, *e.g.* an actuated folding blade, moves or is actuated to fold a portion of said absorbent insert proximal to the front and/or back transversal edges thereof onto itself. In other words, the method preferably comprises a step such as statically folding a portion of said absorbent insert proximal to the front and/or back transversal edges thereof onto itself, preferably in the shape of a substantially U-fold, to form transversal waist barrier(s).

According to an embodiment, the method further comprising the steps of folding an outer nonwoven layer of the front and/or back panels over the folded absorbent insert and joining said folded outer nonwoven layer directly or indirectly to the folded absorbent insert.

According to an embodiment, the method further comprising the step of arranging an elastic material over the folded portion of the folded absorbent insert prior to step of folding an outer nonwoven layer of the front and/or back panels over the folded absorbent insert. Preferably the elastic material is arranged such that said elastic material is sandwiched between the absorbent insert and the outer nonwoven layer. More preferably, the absorbent insert comprises a backsheet and eventually an outer cover on the garment facing surface of the absorbent core, the elastic material being sandwiched between the backsheet and the outer nonwoven layer or the elastic material being sandwiched between the outer cover and the outer nonwoven layer or the elastic material being sandwiched between the backsheet and outer cover and the outer nonwoven layer e.g. in cases where the outer cover is transversally shorter than the backsheet. In other words, the elastic material is directly or indirectly joined to the backsheet, preferably by adhesive, and sandwiched between the backsheet and/or outer cover and the outer nonwoven layer.

According to an embodiment, the method further comprising the step of intermittently applying adhesive, preferably in the machine direction, onto said portion of the absorbent insert proximal to the front and/or back transversal edges.

According to an embodiment, the method further comprising the step of arranging an elastic material over the folded portion of the folded absorbent insert prior to step of folding an outer nonwoven layer of the front and/or back panels over the folded absorbent insert.

According to an embodiment, the method further comprising the step of intermittently applying adhesive onto said portion of the absorbent insert proximal to the front and/or back transversal edges.

According to an embodiment, the method further comprising the step of applying adhesive onto said portion of the absorbent insert proximal to the front and/or back transversal edges in an adhesive pattern, said adhesive pattern comprising at least two areas of adhesive arranged at the transversal extremities of the front and/or back transversal edges, a further area of adhesive extending transversally between said two areas of adhesive, said further area being at a longitudinal distance from the transversal edge, and an adhesive-free area arranged between the transversal edge of the absorbent insert and said further area.

According to an embodiment, the method further comprising the step of compressing the folded portion of the folded absorbent insert, preferably at least the transversal waist barrier(s), once formed prior to the steps of folding an outer nonwoven layer of the front and/or back panels over the folded absorbent insert and joining said folded outer nonwoven layer directly or indirectly to the folded absorbent insert.

According to an embodiment, the method further comprising the step of strand coating the elastic material prior to the step of arranging said elastic material over the folded portion of the folded absorbent insert.

According to an embodiment, the method further comprising the step of compressing the folded portion of the folded absorbent insert once the outer nonwoven layer has been joined directly or indirectly to the folded absorbent insert.

According to an embodiment, the method further comprising the step of mechanically bonding the folded transversal edge of the absorbent insert once the transversal waist barrier(s) has been formed.

In a third aspect the disclosure relates to an apparatus for the manufacture of an absorbent article according to a method as described herein wherein the apparatus comprises:

- a conveying device, preferably a conveyor belt, for conveying an absorbent insert;
- a folding unit for folding a portion of said absorbent insert proximal to the front and/or back transversal edges thereof onto itself, preferably in the shape of a substantially U-fold, to form transversal waist barrier(s);
- a rotating drum and/or a second conveying device, preferably a conveyor belt, for joining said insert to said front and back panels;
- a bonding unit for joining the joining said front and back panels together along a pair of oppositely disposed side seams.

Alternatively or in combination, the disclosure relates to an apparatus for the manufacture of an absorbent article according to a method as described herein wherein the apparatus comprises:

○ a conveying device, preferably a conveyor belt, for conveying an absorbent insert;
○ a bonding unit for applying adhesive and/or for mechanically bonding the patch to the absorbent insert and/or to the front and/or back panels;
○ a patch applying unit for depositing a patch onto the absorbent insert and/or onto the front and/or back panels ;
○ a rotating drum and/or a second conveying device, preferably a conveyor belt, for joining said insert to said front and back panels;
○ a bonding unit for joining the joining said front and back panels together along a pair of oppositely disposed side seams.

According to an embodiment, the apparatus further comprises an elastic applying unit to deposit elastic strand(s) onto the patch, preferably combined with a strand coating device.

According to an embodiment, a pressure roller (46) is arranged to press the folded transversal edge of the absorbent insert (4), said pressure roller (46) being arranged downstream of the folding unit (34) and upstream of the rotating drum (48) and/or second conveyor belt (58) with respect to the machine direction.

According to an embodiment, the folding unit comprises, preferably consists of, a stator, preferably in the form of a folding blade said blade comprising a curvature, said curvature spanning over an angle from 0° to at least 180°, preferably at least 200°, more preferably at least 250°, for example 275°.

According to an embodiment, the folding unit further comprises a pressing blade arranged at a distance from the folding blade with respect to the cross direction and/or vertical direction and arranged to press on absorbent insert at a portion located between the portion of the absorbent insert proximal to the front and/or back transversal edges and the central portion of the absorbent insert.

According to an embodiment, the pressing blade is shorter in length, with respect to the machine direction.

According to an embodiment, the pressing blade comprise a sloped portion at the upstream portion of the pressing blade with respect to the machine direction, said sloped portion extending diagonally downward while extending downstream with respect to the machine direction.

According to an embodiment, the apparatus comprises an adhesive dispensing device arranged upstream of the folding unit with respect to the machine direction, said device being configured to apply adhesive intermittently.

According to an embodiment, a pressure roller is arranged to press the folded transversal edge of the absorbent insert, said pressure roller being arranged downstream of the folding unit and upstream of the rotating drum and/or second conveyor belt with respect to the machine direction.

According to an embodiment, the folding unit comprises a support device to carry the folding blade and/or pressing blade said support device comprising means to move the folding blade and/or pressing blade or comprising means to remove the folding blade and/or pressing blade, preferably in the cross and/or vertical directions (CD,Z).

According to an embodiment, the folding unit comprises an adjustable mechanism to adjust the distance between the pressing blade and the folding blade with respect to the cross direction and/or vertical direction.

According to an embodiment, the folding unit comprises two folding blades, preferably each with a pressing blade, said folding blades being arranged at each end of the conveyor belt with respect to the cross direction, the folding blades being substantially identical in dimensions and shape.

All embodiments herein can be taken alone or in combination as the features herein are typically interchangeable.

## BRIEF DESCRIPTION OF THE FIGURES

**FIG.** 1 is a top planar view of an absorbent article according to an embodiment of the present disclosure.

**FIG.** 2 is a top planar view of an absorbent article according to an embodiment of the present disclosure.

**FIG. 3** is a partial cross-section of an absorbent article according to an embodiment of the present disclosure.

**FIG. 4** is a top planar view of an absorbent article according to an embodiment of the present disclosure.

**FIG. 5** is a side view, or profile view, of the apparatus for the manufacture of absorbent articles according to an embodiment of the present disclosure.

**FIG. 6** is a view in perspective of an isolated component, the folding blade, of the apparatus of FIG. 5.

**FIG. 7** is a view in perspective of an isolated component, the folding unit, of the apparatus of FIG. 5.

**FIG. 8** is a view in perspective of isolated components, the first conveyor belt with the folding unit, of the apparatus of FIG. 5.

**FIG. 9** is a cross section of the folding blade at the downstream portion with respect to the machine direction MD.

**FIG.** 10 is a top planar view of an absorbent article according to an embodiment of the present disclosure.

**FIG. 11A** is a top planar view of an absorbent article according to an embodiment of the present disclosure prior to the folding of the portion of said absorbent insert proximal to the back transversal edge.

**FIG. 11B** is a top planar view of an absorbent article according to FIG. 11A after folding of the portion of said absorbent insert proximal to the back transversal edge.

**FIG. 12** is a top planar view of an absorbent article according to an embodiment of the present disclosure.

**FIG. 13** is a top planar view of an absorbent article according to an embodiment of the present disclosure.

**FIG. 14** is a partial cross-section of an absorbent article according to an embodiment of the present disclosure.

**FIG. 15** is a partial cross-section of an absorbent article according to an embodiment of the present disclosure.

**FIG. 16** is a partial top planar view of an absorbent article according to an embodiment of the present disclosure.

**FIG. 17** is a top planar view of an absorbent article according to an embodiment of the present disclosure.

**FIGS. 18A-18C** are cross section views of precursors according to an embodiment of the present disclosure.

**FIG. 18D** is a cross section view taken about section line L1 in FIG.17, or equally FIG.19 for that matter.

**FIG. 19** similarly to Fig. 17 is a top planar view of an absorbent article according to an embodiment of the present disclosure.

## DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed disclosure. However, it is to be understood that the value to which the modifier "about" refers is itself also specifically disclosed.

The expression "% by weight" (weight percent), here and throughout the description unless otherwise defined, refers to the relative weight of the respective component based on the overall weight of the formulation.

The recitation of numerical ranges by endpoints includes all numbers and fractions subsumed within that range, as well as the recited endpoints unless otherwise stated.

As used herein, the "skin facing", "body-facing" or "bodyside" surface means that surface of the article or component which is intended to be disposed toward or placed adjacent to the body of the wearer during ordinary use, while the "outward", "outward-facing" or "garment-side" or "garment facing" surface is on the opposite side, and is intended to be disposed to face away from the wearer's body during ordinary use. Such outward surface may be arranged to face toward or placed adjacent to the wearer's garments or undergarments when the absorbent article is worn.

As used herein, the term "absorbent article" refers to disposable devices such as infant or adult diapers or pads, pants, training pants, and the like which are placed against or in proximity to the body of the wearer to absorb and contain the various exudates discharged from the body. Typically these articles comprise a topsheet, backsheet, an absorbent core and optionally an acquisition system (which may be comprised of one or several layers) and typically other components, with the absorbent core normally placed between the backsheet and the acquisition system or topsheet.

The absorbent articles of the disclosure will be further illustrated in the below description and in the Figures, though all embodiments described herein may equally be applied onto absorbent articles in the form of pants (or even in the form of feminine hygiene articles such as menstrual pants and/or slips/panties). Nothing in this description should be however considered limiting the scope of the claims unless explicitly indicated otherwise. Unless indicated otherwise, the description refers to the dry article, i.e. before use and conditioned at least 24 hours at 21° C.+/-2° C. and 50+/-20% Relative Humidity (RH).

A "nonwoven web" as used herein means a manufactured sheet, web or batt of directionally or randomly oriented fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yarn). Nonwoven webs can be formed by many processes such as meltblowing, spunbonding, solvent spinning, electrospinning, carding and airlaying. The basis weight of nonwoven webs is usually expressed in grams per square meter (g/m2 or gsm).

The terms "joined" or "associated" or "bonded" or "attached", as used herein, encompasses configurations whereby an element is directly secured to another element by affixing the element directly to the other element, and configurations whereby an element is indirectly secured to another element by affixing the element to intermediate member(s) which in turn are affixed to the other element. The terms further include embodiments in which a pocket or other connector is formed in or attached to an area of the absorbent article. Further, these terms include configurations in which the elements are removably, or non-removably, joined, bonded, or attached. For example, wherein an element is described as "joined" within the configuration, it may be either removably joined or non-removably joined unless otherwise specified or evident from the context.

The terms "comprise," "comprising," and "comprises" are open ended terms, each specifies the presence of what follows, e.g., a component, but does not preclude the presence of other features, e.g., elements, steps, components known in the art, or disclosed herein. These terms based on the verb "comprise" should be read as encompassing the narrower terms "consisting of" which excludes any element, step, or ingredient not specified and "consisting essentially of" which limits the scope of an element to the specified materials or steps and those that do not materially affect the way the element performs its function. Any preferred or exemplary embodiments described below are not limiting the scope of the claims, unless specifically indicated to do so. The words "typically", "normally", "advantageously" and the likes also qualify elements which are not intended to limit the scope of the claims unless specifically indicated to do so.

By "absorbent material" it is meant a material which has some absorbency property or liquid retaining properties, such as SAP, cellulosic fibers as well as synthetic fibers, most preferably is selected from the group consisting of SAP, cellulose (or cellulosic) fibers, and mixtures thereof. Herein, absorbent materials in the form of fibrous absorbent materials have been found to be useful. These fibrous absorbent materials can comprise or consist of natural fibers, e.g. cellulosic fibers as well as synthetic fibers. Typically, glues used in making absorbent cores have no absorbency properties and are not considered as absorbent material.

As used herein, the term "absorbent core" refers to the component or components of the article having the most absorbent capacity and comprising an absorbent material and optionally a core wrap enclosing the absorbent material. The term "absorbent core" does not include the acquisition-distribution system or layer or any other component of the article which is not either integral part of the core wrap or placed within the core wrap. The core may consist essentially of, or consist of, a core wrap, absorbent material as defined below and glue enclosed within the core wrap.

The values indicated herein are measured according to the methods indicated herein below, unless specified otherwise. All measurements are performed at $21 \pm 2°$ C. and $50 \pm 20\%$ RH, unless specified otherwise. All samples should be kept at least 24 hours in these conditions to equilibrate before conducting the tests, unless indicated otherwise. All measurements should be reproduced on at least 4 samples and the average value obtained indicated, unless otherwise indicated.

Absorbent articles (1) herein are for personal hygiene, preferably a disposable pant, and comprise:

- a substantially transversely extending front panel (2);
- a substantially transversely extending back panel (3); and
- a substantially longitudinally extending absorbent insert (4) joined to each of said front and back panels (2, 3), said absorbent insert (4) comprising front and back transversal edges and left and right longitudinal edges connecting the front and back transversal edges to form a perimeter thereof; and wherein said insert (4) comprises an absorbent core (8);

wherein the front and back panels (2, 3) are joined together to define a pair of side seams.

The article comprises a transversal waist barrier (5) proximal to the front and/or back transversal edges of the absorbent insert (4) wherein said barrier (5) comprises a plurality of liquid retarding layers wherein said plurality of liquid retarding layers comprise at least two layers selected from the group consisting of hydrophobic nonwovens, films, and/or strands (preferably wherein at least one of said at least two layers is a film, more preferably a substantially liquid impermeable film); and wherein at least a majority of the waist barrier (5) comprises at least two of said plurality of liquid retarding layers and wherein said at least two of said plurality of liquid retarding layers are un-joined at one or more locations so that said layers are separated from one another at said location(s) by a gap (G), and preferably wherein said one or more locations together correspond to a majority of a total surface of said waist barrier (5). Advantageously barriers formed this way allow for reduced risk of leakage in use, yet being effective in production at high speeds that may exceed 850 pieces per minute. Such a gap or gaps are generally formed by ensuring that when the liquid retarding layers (e.g. hydrophobic nonwovens as described herein, like between inner and outer layers of the front and/or back panel(s) as described herein) are joined together, they are joined at discrete locations (e.g. stripes, patches, swirls or the like of adhesive, mechanical bonding, or other suitable joining means such as using strand-coated elastics as the joining means as described in more detail herein) so that un-joined regions exist where the layers can be in close proximity to each other and/or in contact but are able to separate upon application of a force that is generally smaller in magnitude to that required to separate joined layers, and may do so without tear or damage.

The core (8) may comprise one or more channels (13), preferably substantially free of absorbent material; each said channel generally being circumscribed by absorbent material. As also described in more detail herein the absorbent core may comprise a core wrap to enclose the absorbent material, and the channel(s) may be formed by joining top and bottom layers of said core wrap together (any suitable joining technique may be used such as via adhesive and/or mechanical bonding such as heat and/or pressure bonding, ultrasonic bonding and the like). The core wrap may be made of any suitable nonwoven such as spunbond and/or meltblown nonwoven; and/or a carded nonwoven such as a carded air-through-bonded nonwoven or a carded calender-bonded nonwoven; such nonwovens particularly preferred in case of absence of an optional acquisition distribution layer between the core/core wrap and topsheet.

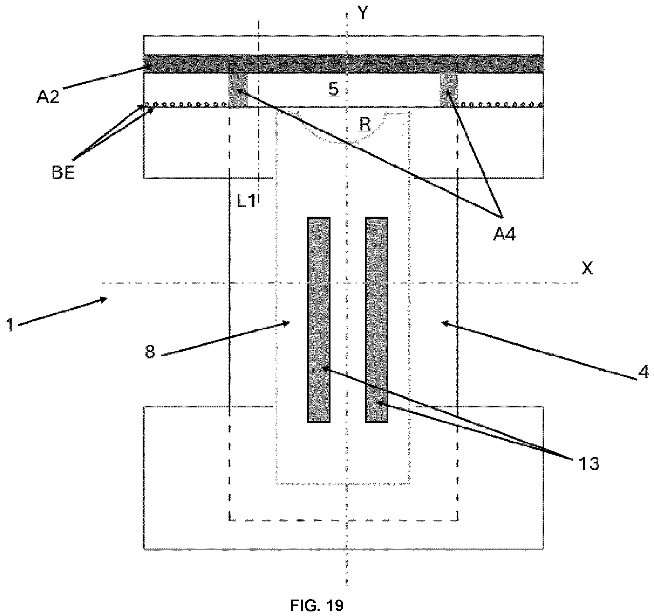

**The** channel(s) herein may have a width (generally extending substantially parallel to the transverse x axis or centerline) of from 5mm to 30mm, preferably from 10mm to 25mm. The channel(s) may have a length (generally extending substantially parallel to the longitudinal y axis or centerline) that is from 10% to 80%, preferably from 15% to 75%, even more preferably from 20% to 70%, of a total length of the absorbent core (8) [when multiple disconnected channels along the y axis are present the % length is the sum of the length of each said channels divided by the total absorbent core length]. **The** channel(s) may have a variety of shapes depending on the intended purpose for example can have any of the shapes described in EP3453368. Other suitable shapes are described in EP3342386 or WO2012/170778 (Rosati et al., see also WO2012/170779, WO2012/170781 and WO2012/1708008). Fig. 19 further illustrates exemplary channels. Advantageously channels according to the above embodiment may aid to promote fast liquid distribution therealong thus improving acquisition speed performance of the article whilst mitigating against risks of leakage.

**The** core (8) may comprise a recessed region (R) proximal and/or adjacent to the barrier (5). Fig.19 exemplifies an embodiment of such recessed region. **The** recessed region may be a region that has a lower basis weight of absorbent material compared to the rest of the absorbent core (8) or may even be substantially free of absorbent material; alternatively albeit less preferred, the recessed region may comprise compressed absorbent material such as by embossing or other similar technique. The recessed region (R) may be partly enclosed by absorbent material [or of higher basis weight thereof] such that said absorbent material of the core (8) defines and/or delimits at least a portion of said recessed region (R). The recessed region may be in the form of an arc or similar geometry, including for example with curved and/or linear edges forming a substantial C-shape, U-shape, or V-shape; all also contemplated and within the meaning herein. Advantageously such recessed region may not only allow to increase the volume available for the pocket formed by the barrier but further allows the barrier to better form a standing gather and thus form a larger opening for exudate collection therein.

The recessed region (R) may be centrally located relative to the absorbent core, as exemplarily shown in one form thereof in Fig.19. Indeed, the gluteal cleft is centrally located on the body, hence the central location of the recessed region being preferred, moreover, the generally lower thickness of the recessed region results in the pocket formed under the barrier not only having more volume to more efficiently gather bodily exudates as explained above but further when a user wears the absorbent article and subsequently puts pressure on the absorbent core by means of sitting, lying down or any other way, the recessed region will contribute to being exposed to a lower pressure compared to the surrounding areas of the article which may result in that the surrounding parts of the article will have a pressure receiving function, as a consequence, the bodily exudates will flow into the pocket.

When both a recessed region (R) and channel(s) (13) are present, it is preferable that the largest width of the recessed region is greater than the width of the channel(s), preferably is at least 150% greater, more preferably at least 200% greater, even more preferably from 2.5 to 9, even more preferably from 4 to 9, times greater than the width of the channel(s) (13). By means of example, if the channel(s) have a width of 10mm, the largest width of the recessed region (R) may be at least 15mm, preferably 20mm, even more preferably from 25mm to 90mm, even more preferably from 40mm to 90mm. Advantageously this may allow for maximised pocket volume and pressure relief without compromising on acquisition speed and absorption capacity.

Preferably, when both a recessed region (R) and channel(s) (13) are present, they are mutually separated by absorbent material generally such that the channel(s) are not directly connected to the recessed region but are rather spaced apart therefrom. The distance between the channel(s) and the recessed region may be greater or equal to the width of the channel(s). Advantageously, in this manner risk of exudate leakage, especially urine, is reduced as separation by the absorbent material prevents urine that is being effectively ducted along the channels from entering the pocket.

In a preferred embodiment, the front and back panels (2, 3) are refastenably joined together to define the pair of side seams such that the seams can be opened and re-closed, preferably wherein the seams are maintained in closed state by one or more cooperating male and female fasteners preferably hook and loop fasteners respectively. Advantageously, the barrier described herein is particularly effective when combined with a refastenable pant in that exudates collected in the barrier can be wrapped during removal from the subject and fastened in a closed/enclosed state for disposal in an easy manner.

The article may have a Seepage Index of less than 35%, preferably of less than 30%, preferably less than 25%, more preferably less than 20%, even more preferably less than 10%, even more preferably from 0% to 5%, according to the Seepage Index Method herein. Preferably wherein the transversal waist barrier (5) is a laminate comprising an elastic film or a plurality of elastic strands sandwiched between said hydrophobic nonwoven that can be a single hydrophobic nonwoven that is folded to sandwich said elastic film or strands, or a plurality of hydrophobic nonwovens sandwiching said film or strands therebetween; and preferably said elastic film or stands being sandwiched between two hydrophobic nonwovens (or layers). Advantageously articles arranged in this manner have been found to significantly reduce the amount of leakage of exudates and in particular allow for further absorbent core utilisation by wicking that increases the time elapsed before a pocket could be completely filled with free-flowing exudates that would otherwise increase risk of leakage. Moreover, without wishing to be bound by theory, Seepage Index is a good indicator of the ability of the barrier to resist to leakage even in the most challenging of conditions such as at an inclined position and after being subjected to movement (e.g. by movement and wearing over a prolonged period of time). Absorbent articles herein are particularly performant in their ability to retain liquid and reduce leakage compared to the prior art articles.

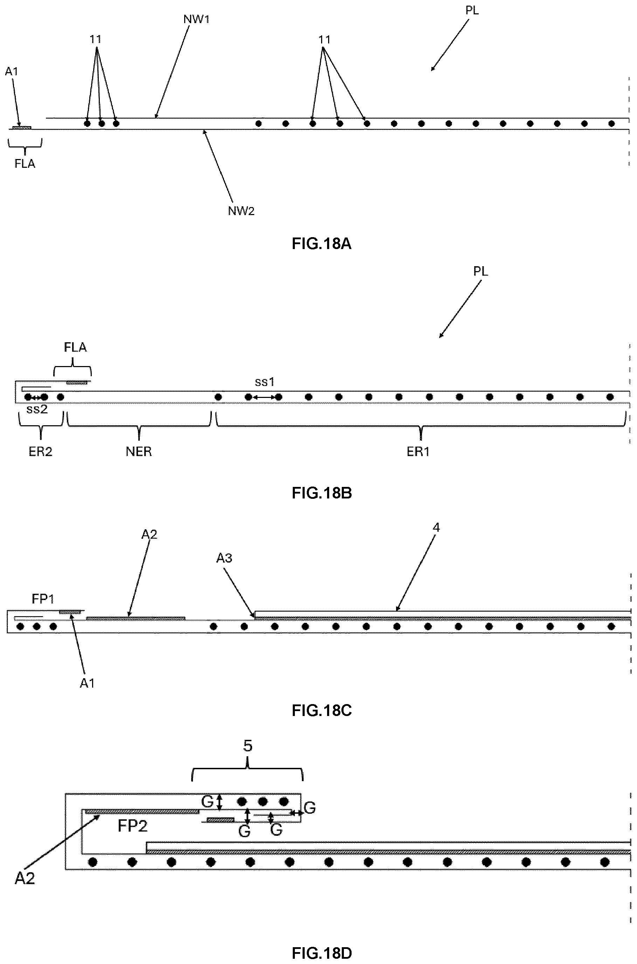

Preferably, the barrier (5) comprises three or more of said liquid retarding layers (preferably each being a hydrophobic nonwoven as described herein) at least over a portion of said barrier (5), an exemplary embodiment is illustrated in Fig.18D. Advantageously structures with such multiple layers in combination with the described gap allows for an effective exudate barrier even when not utilising a substantially liquid impermeable film.

The waist barrier (5) preferably comprises at least two hydrophobic nonwovens intermittently joined together by adhesive, preferably by one or more elastic strands that are strand-coated with adhesive and sandwiched therebetween. Advantageously this allows to maximise the un-joined region(s) that generally form the gap(s) referred to herein and hence minimising contact between layers. For sake of clarity, the nonwovens need not be solely or exclusively joined together by said strand-coated elastics but further joining means such as further adhesive areas or discrete bonding such as mechanical bonding may also be used yet preferably taking care that not the entire respective surfaces of the nonwovens are joined together so as to maintain un-joined regions within the barrier construct.

The front and/or back panels (2, 3) preferably comprise a plurality of elastic strands (11) sandwiched between two or more hydrophobic nonwoven layers, said nonwoven layers comprising at least an inner nonwoven (NW1) and an outer nonwoven (NW2) that are substantially congruent with each other to form a front and/or back panel laminate (PL); and wherein the front and/or back panel laminate (PL) is folded onto itself to form the barrier (5) and such that both the inner and outer nonwovens (NW1, NW2) are folded at one or more positions. Figs.18A-D are particularly representative of such exemplary embodiments. "Substantially congruent" as used herein above does not require said layers to be entirely congruent, it is rather advantageous, encompassed in its meaning, and/or preferred that one of the layers (preferably the inner nonwoven layer (NW1)) is somewhat shorter than the other (preferably the outer nonwoven layer (NW2)) such that a flange (FLA) is formed that can be folded and joined to the said other layer (preferably the outer nonwoven layer (NW2)) generally to securely contain the elastics (11) securely with limited risk of irritation to the wearer in case of snap-back, yet generally retaining a portion of said other layer within the fold so as to create a further buffer to restrict leakage of exudates.

Preferably, the barrier (5) is arranged such that the folded outer nonwoven (NW2) comes into direct contact with the skin of the wearer when the article is in use. At least a portion of the inner nonwoven (NW1) may be in direct contact with the skin of the wearer when the article is in use, typically in at least some regions of the front and/or back panels other than the barrier (5).

At least the inner nonowoven (NW1) is preferably a multi-layer nonwoven comprising at least two, preferably at least three, layers selected from a spunbond nonwoven and a meltblown nonwoven, more preferably said nonwoven being selected from a spunbond-meltblown nonwoven, a spunbond-meltblown-spunbond nonwoven, and a spunbond-meltblown-meltblown-spunbond nonwoven. Such laminates are advantageous in terms of mechanical properties but may have drawbacks in terms of softness.

The outer nonwoven (NW2) is a nonwoven that is the same or different to the inner nonwoven (NW1), preferably the outer nonwoven (NW2) comprising, more preferably consisting of, one or more spunbond nonwoven layers. Advantageously supunbond nonwoven layers that are generally free of meltblown layers are softer and can provide a better feel sensation for the caregiver handling the article when placing it on a subject as well as reducing the risk of irritation at the waist of the wearer when combined with the novel barrier folding described herein. Mechanical integrity of the overall laminate is nevertheless attainable by the combination thereof with the multilayer inner nonwoven (NW1) described above to achieve a sweetspot in terms of mechanical performance, softness and cost.

As exemplary shown in Figs.18A-D, the front and/or back panels (2, 3) preferably comprise a first elastic region (ER1) and a second elastic region (ER2) wherein the first and second elastic regions (ER1, ER2) each comprise one or more elastic strands (11), and wherein the first and second elastic regions (ER1, ER2) are separated by a non-elastic region (NER), wherein the one or more positions of the fold(s) substantially correspond the non-elastic region (NER). Preferably, the first elastic region (ER1) comprises a plurality of spaced-apart elastic strands (11) having one or more first strand spacings (ss1) and wherein the second elastic region (ER2) comprises one or more elastic strands (11).

The second elastic region (ER2) preferably comprises a plurality of spaced-apart elastic strands (11) having one or more second strand spacings (ss2) and wherein the first and second strand spacings are different, preferably wherein the first strand spacing(s) (ss1) is greater than the second strand spacing(s) (ss2), more preferably wherein the barrier (5) comprises said second elastic region (ER2). Advantageously this aids to create a tension differential that may help the barrier (5) to stand up to form a transversal up-standing gather or cuff.

Preferably, at least one of the elastic strands of the second elastic region (ER2) is coloured so that the barrier (5) is visually discernable from the rest of the article. This may advantageously aid a caregiver to identify the front and back orientation of the article to put onto the subject, especially in case of asymmetric positioning of the elastics in the front vs back and/or the presence of the barrier (5) at only one of the front or back panels.

In a highly preferred embodiment, the outer nonwoven (NW2) is joined to the inner nonwoven (NW1) at a first fold position (FP1), and wherein the inner nonwoven (NW1) is joined to itself at a second fold position (FP2), preferably wherein the first fold position (FP1) and the second fold position (FP2) are different, more preferably wherein the first and second fold positions (FP1, FP2) do not overlap.

The outer nonwoven may be joined to the inner nonwoven at the first fold position (FP1) preferably by a first adhesive (A1). The inner nonwoven (NW1) may be joined to itself at a second fold position (FP2) preferably by a second adhesive (A2). The absorbent insert (4) may be joined to the inner nonwoven (NW1) by a third adhesive (A3). The first, second and third adhesives (A1, A2, A3) may be the same or different. Preferably at least the second adhesive (A2) (and the fourth adhesive (A4) as described below, when present) is hydrophobic, which generally can advantageously help to prevent low viscosity exudates from penetrating out of the pocket nonwoven materials. The adhesive, such as the hydrophobic adhesive, may be a hot melt adhesive. To determine whether a hot melt adhesive is hydrophobic, a portion of the hot melt adhesive is molten and spread on an even, horizontal surface, such as a table, to form a film. Then a drop of water is applied on the film and the contact angle is determined, as is well known in the art. The adhesive is hydrophobic, if the contact angle is of more than 90°.

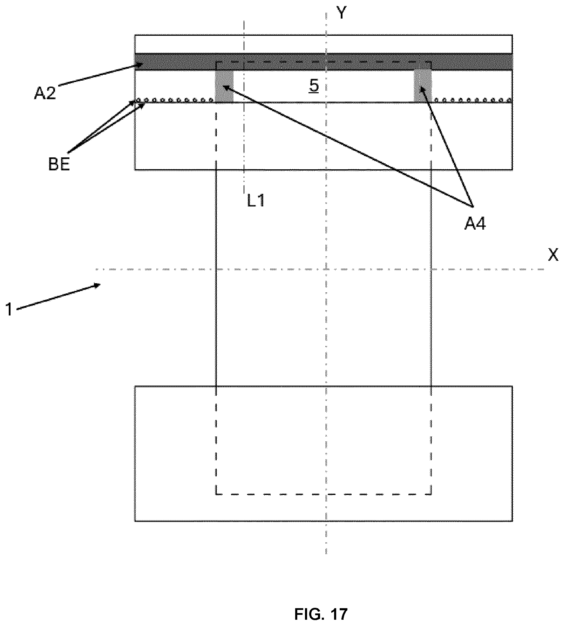

Preferably, as illustrated in Fig. 17, the second adhesive (A2) is applied continuously in a transverse direction (i.e. substantially parallel to the transverse axis x). A further fourth adhesive (A4) is preferably applied in a lengthwise direction (i.e. substantially parallel to the longitudinal axis y) of the barrier (5), said fourth adhesive (A4) being applied discontinuously in a transverse direction such that an un-joined pocket opening is formed therebetween for collecting and/or retaining exudates therein, preferably said fourth adhesive (A4) being positioned outboard and/or peripheral to the said pocket opening.

In order to limit the use of adhesive, discrete bonding elements (BE) may be used to fasten the folded outer nonwoven (NW2) to the inner nonwoven (NW1) at positions outboard of the fourth adhesive (A4). Advantageously this allows for process stability yet reducing cost and waste. The discrete bonding elements may be formed by intermittent application of adhesive or more preferably by mechanical bonding techniques as are known in the art such as pressure and heat bonding, ultrasonic bonding and the like.

In an embodiment, a maximum distance (d2max) between a terminal edge (TE) of said waist barrier (4) corresponding to and/or forming a pocket opening and a closest terminal edge (TEa) of the absorbent core (8) is less than 20 mm, preferably less than 17 mm, even more preferably from 0mm to 15 mm.

In a highly preferred embodiment, a capillary acceleration sheet is comprised along substantially an entire maximum distance (d2max), preferably along the entire said maximum distance, such that a fluid communication bridge is formed between at least the barrier (5) and the absorbent core (8). Advantageously the capillary acceleration sheet may thus enable wicking of the body exudates therealong and towards the absorbent core that may then absorb the exudates even against gravity as the case may be depending on body position of the wearer.

The capillary acceleration sheet is preferably a nonwoven, more preferably the nonwoven comprising, or consisting of, a carded air-through-bonded nonwoven or a spunbond nonwoven. Most preferred are nonwovens comprising an embossment pattern and/or apertures, hence in such embodiments the nonwoven comprises an embossment pattern and/or apertures. The capillary acceleration sheets herein may be comprised (or even formed entirely) of the topsheet of the absorbent article that is typically comprised by the insert, alternatively or in addition it may be an acquisition distribution layer that generally protracts so as to extend substantially along the entire distance d2max. Advantageously not only does this allow for a cost-effective wicking layer but further increases surface area that limits exudate gliding (that may exacerbate the risk of leakage) whilst maintaining desired wicking capabilities.

In a highly preferred embodiment, the absorbent core (8) comprises cellulose fibers and said cellulose fibers are comprised at a level of at least 10%wt, preferably at least 12%wt, more preferably at least 15%wt, even more preferably at least 20%wt, even more preferably from 22%wt to 40%wt, by weight of the absorbent core. Advantageously the cellulose further acts as wicking material that aids to carry exudates from the pocket into the core by capillary action before being absorbed by superabsorbent polymer particles as described herein. Interestingly, the combination of a capillary acceleration sheet as described herein above in combination with an absorbent core comprising the above recited amounts of cellulose fibers allow for a synergistic cooperation that enhances the exudate draining properties of pockets described herein.

As will also be discussed in more detail herein below, in a most preferred embodiment the transversal waist barrier (5) comprises a film (typically joined to one or two nonwovens as described in more detail in embodiments herein). The film preferably extends over the majority of the waist barrier (5) or even has a total surface area that substantially corresponds to that of the barrier (5). It being understood that in embodiments herein where the barrier (5) is formed by folding of the insert the film surface area compared to the barrier (5) surface area may be greater than if the barrier (5) is formed by means of a patch in other embodiments herein; yet maintaining similar advantages being better leakage prevention especially at different body positions, sweat and the like that a wearer may subject the absorbent article to.

In an embodiment, a maximum distance (d1max) between a terminal transversal edge (TT) of said waist barrier (4) corresponding to and/or forming a closed-end of said pocket and a closest terminal edge (TEa) of the absorbent core (8) is less than 80 mm, preferably less than 60 mm, even more preferably less than 50 mm, even more preferably from 0mm to 20mm. Advantageously positioning the core not too far from the terminal transversal edge allows for pocket draining by the core when filled with exudates.

In an embodiment, the ratio d1max/ d2max is greater than 2.5, preferably greater than 3.0, even more preferably from 3.5 to 50. Advantageously this allows for a balance between pocket size for capturing exudates therein and ability for exudates to be drained from the pocket to limit leakage that could arise for example upon application of pressure if not drained and absorbed by the core.

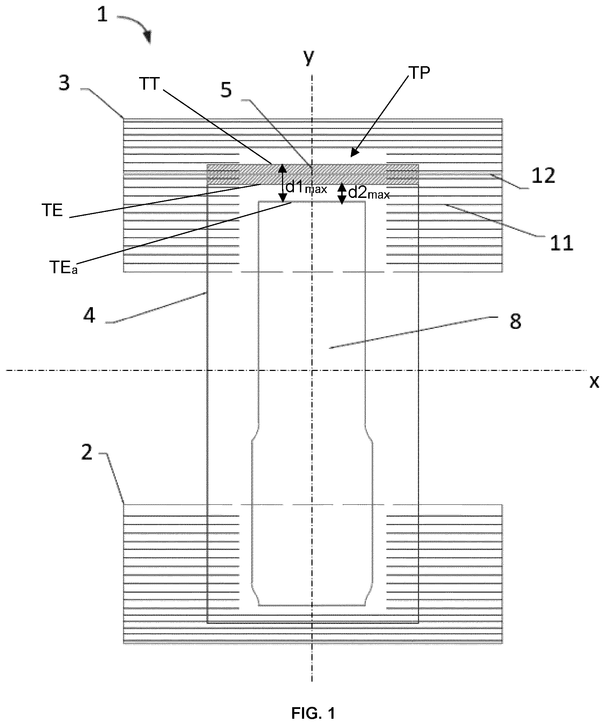

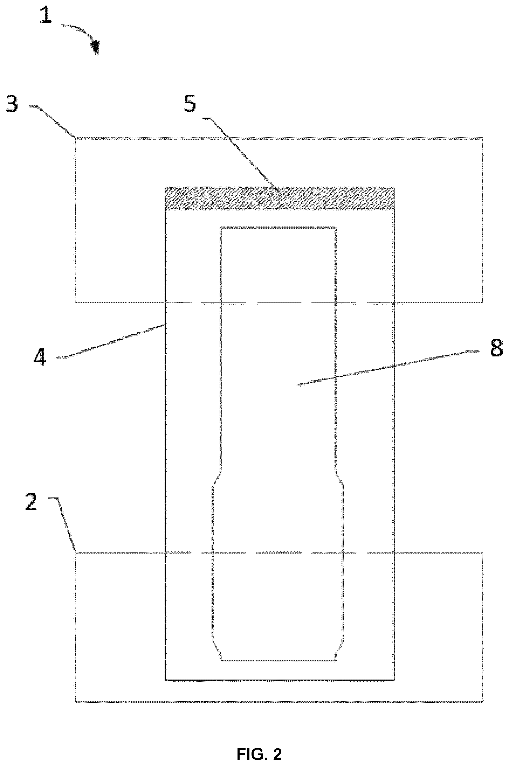

In an embodiment, as exemplified in Fig.1 to Fig.3, absorbent articles herein are for personal hygiene, preferably in the form of a pant, the absorbent article comprising: a substantially transversely extending front panel (2); a substantially transversely extending back panel (3); and a substantially longitudinally extending absorbent insert (4) joined to each of said front and back panels (2, 3), said absorbent insert (4) comprising front and back transversal edges and left and right longitudinal edges connecting the front and back transversal edges to form a perimeter thereof (generally wherein the left and right longitudinal edges extend substantially parallel to the longitudinal axis y and are oppositely disposed such that said longitudinal axis y extends therebetween, and the front and back transversal edges extend substantially parallel to the transversal axis x, and typically substantially perpendicular to the longitudinal axis y, and are oppositely disposed such that said transverse axis x extends therebetween); the absorbent insert (4) preferably comprising an absorbent core (8); and wherein the front and back panels (2, 3) are joined together to define a pair of side seams, and wherein at least a portion of said absorbent insert (4) proximal to the front and/or back transversal edges thereof comprises a transversal waist barrier(s) (5) comprising a substantially liquid impermeable film layer, preferably wherein said transversal waist barrier (5) is an up-standing barrier generally arranged to block substantially liquid exudates from flowing therethrough. Advantageously, this arrangement allows for an absorbent article with superior leakage prevention particularly for substantially liquid exudates such as urine and/or liquid stool.

Generally, the substantially liquid impermeable film layer may be an integral component of at least one element or portion of the absorbent article such as a backsheet (7) thereof typically such that no additional and/or separate layer, element and/or material is incorporated to form the transversal waist barrier(s) (5). Advantageously this allows to reduce waste and/or cost associated with incorporating separate elements to create the transversal waist barrier(s).

The backsheet (7) is generally that portion of the absorbent article positioned adjacent the garment-facing surface of the absorbent core (8) and which prevents the exudates absorbed and contained therein from soiling articles such as bedsheets and undergarments. The backsheet (7) is typically impermeable to liquids (e.g. urine). The backsheet (7) may for example be or comprise a thin plastic film such as a thermoplastic film having a thickness of about 0.012 mm to about 0.051 mm. Preferably the basis weight of the backsheet (7) is less than or equal to 16 g/m$^2$, more preferably from 10 g/m$^2$ to 14 g/m$^2$. Exemplary backsheet films include those manufactured by Tredegar Corporation, based in Richmond, Va., and sold under the trade name CPC2 film. Other suitable backsheet materials may include breathable materials which permit vapors to escape from the absorbent article while still preventing exudates from passing through the backsheet (7). Exemplary breathable materials may include materials such as woven webs, nonwoven webs, composite materials such as film-coated nonwoven webs, microporous films such as manufactured by Mitsui Toatsu Co., of Japan under the designation ESPOIR NO and by Tredegar Corporation of Richmond, Va., and sold under the designation EXAIRE, and monolithic films such as manufactured by Clopay Corporation, Cincinnati, Ohio under the name HYTREL blend P18-3097. Some breathable composite materials are described in greater detail in PCT Application No. WO 95/16746 published on Jun. 22, 1995 in the name of E. I. DuPont; U.S. Pat. No. 5,938,648 to LaVon et al., U.S. Pat. No. 4,681,793 to Linman et al., U.S. Pat. No. 5,865,823 to Curro; and U.S. Pat. No. 5,571,096 to Dobrin et al, U.S. Pat. No. 6,946,585B2 to London Brown.

In a preferred embodiment the backsheet (7) is breathable. Breathable backsheet herein typically means that it comprises micro-openings sized to allow at least some water vapour to permeate through the backsheet that typically comprises a film such as a polyethylene (PE) film.

Preferably a backsheet (7) is breathable when the water vapour transmission rate (WVTR) of the backsheet is at least 500 grams/m2 - 24 hours, preferably at least 1,000 grams/m2 - 24 hours, even more preferably from 1,200 grams/m2 - 24 hours to 2,500 grams/m2 - 24 hours, even more preferably from 1,500 grams/m2 - 24 hours to 2,100 grams/m2 - 24 hours, as measured according to ASTM D6701-21.

The backsheet (7) may be joined to the topsheet (6), the absorbent core (8) or any other element of the absorbent article by any attachment means known in the art. Suitable attachment means are described above with respect to means for joining the topsheet to other elements of the article. For example, the attachment means may include a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minn. and marketed as HL-1620 and HL 1358-XZP. Alternatively or in combination, the attachment means may comprise heat bonds, pressure bonds, ultrasonic bonds, dynamic mechanical bonds, or any other suitable attachment means or combinations of these attachment means as are known in the art.

The absorbent core (8) of the absorbent article may comprise one or more core layers. As explained, the absorbent article may comprise an acquisition distribution system, which will typically consist of one or more layers. Most typically, the layers are arranged above the core layer. Hence, a number of layers can be arranged between the topsheet and the backsheet. The skilled person will usually have no difficulty in distinguishing between these layers. In case of doubt, a core layer can be identified as being a layer which is generally less permeable than a layer forming part of the acquisition-/distribution-system.

Permeability generally refers to the quality of a porous material that causes it to a lower liquid or gases to pass through it. Hence, the layers of the acquisition distribution system should generally be more permeable than the layers of the core system as these layers are meant to distribute liquid to the absorbent core, where the liquid is ultimately stored.

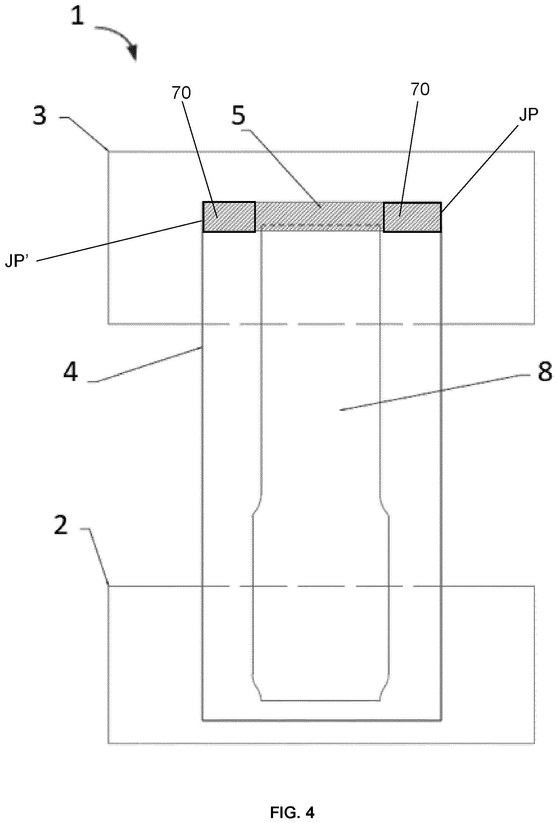

In an embodiment, as exemplified in Fig. 4, the transversal waist barrier(s) (5) overlap at least a portion of an absorbent core (8) of the absorbent insert (4) typically such that the exudates collected and/or retained by the transversal waist barrier(s) (5) are substantially dehydrated and/or absorbed by the neighbouring absorbent core (8) surface(s). Advantageously this allows for reduced leakage risks of highly liquid exudates like urine or liquid stool such as diarrhoea (or other liquid stool that is generally and commonly observed e.g. with new-born babies).

Preferably the front and back panels (2, 3) are separate and connected to each other by the absorbent insert (4) acting as a bridge element to form a substantially H-shaped pant. Advantageously this allows to omit cutting/shaping of panels in a crotch region of the article thus reducing waste and/or limiting the amount of panel material in view of its absence in the crotch region also helping to reduce waste and cost.

The absorbent insert (4) may be joined to the front and back panels (2, 3) by adhesive and/or mechanical bonding such as ultrasonic bonding, pressure bonding, and/or thermal bonding and the like. In a preferred embodiment the absorbent insert (4) is joined to the front and back panels (2, 3) by a discontinuous pattern of adhesive. Advantageously this may help to retain softness and pliability of the laminated structures and reduce stiffness when worn.

In an embodiment, the substantially liquid impermeable film layer comprises, preferably consists of, a backsheet (7) of the absorbent insert (4) and preferably comprises a material comprising, or consisting of, a polyester, more preferably said material being selected from the group consisting of polyethylene, polylactic acid, and polypropylene. Advantageously such transversal waist barrier(s) are not only impermeable to a much higher degree than hydrophobic nonwovens but further are formed by using a component already present in the absorbent article and hence does not require adding any further materials that would impact waste and/or cost.

Preferably, the film is substantially liquid impermeable and said film forms an inner wall of the barrier (5) such that it remains exposed and is able to come into direct contact with exudates upon a voiding event, preferably wherein said film is positioned at a garment facing side of said barrier (5) and facing the body facing side of the topsheet. The film may hence form an internal wall structure of the barrier. Advantageously this arrangement allows for a cost-effective solution limiting material use of the barrier yet ensuring the avoidance of irritation to the skin of a wearer whilst maintaining exceptional leakage prevention.

A nonwoven layer as described herein may be folded over a terminal edge of the film and joined to said film at some distance from said edge (e.g. at a position on the inner wall of the barrier); or extends beyond the film. Advantages include protection for the wearer and limiting the risk of direct skin contact with the film yet reducing the amount of material used.

Preferably, the barrier comprises a nonwoven layer over at least a substantial portion of the inner wall of the barrier (for example the full inner wall of the barrier may comprise such nonwoven layer when a nonwoven-film-nonwoven laminate is used having the film sandwiched between two nonwoven layers). Advantageously, the fibrous network of the nonwoven aids to slow down exudates as they travel along the inner wall of the barrier upon application of pressure and hence aids in limiting leakage from the barrier compared to the generally smooth surface of impermeable films.

The barrier may comprise nonwovens selected from carded nonwovens having absorbent fibers such as super absorbent fibers (or SAF) generally having absorbent properties such as those known from super absorbent particles (SAP) but in fibrous form instead of particles; or may comprise nonwovens impregnated with an amount of super absorbent particles (SAP). Advantageously this may aid to dehydrate liquid stool or absorb urine that is trapped by the said barrier.

In an embodiment, the font and/or back panels (2, 3) are elastic and comprise an inner nonwoven layer (9), an outer nonwoven layer (10), and an elastic material (11) sandwiched therebetween, wherein the elastic material is selected from the group consisting of an elastic film and a plurality of elastic strands. Preferably, the outer nonwoven layer (10) is wider than the inner nonwoven layer (9) (generally in a direction substantially parallel to the longitudinal axis y) and is folded over said inner nonwoven layer (9) such that it overlaps the portion of said absorbent insert (4) proximal to the front and/or back transversal edges, preferably such that said outer nonwoven layer (10) overlaps both a portion of the backsheet (7) and a portion of the topsheet (6) that remains exposed from said one or more transversal waist barrier(s) (5) such to form a flange ($F_L$). Advantageously this allows to cover the backsheet with nonwoven so that the backsheet does not come into contact with the skin of a wearer which could otherwise result in skin irritations and/or less comfort.

According to an embodiment, the article comprises a transversal waist barrier (5) proximal to the front and/or back transversal edges of the absorbent insert (4) wherein said barrier (5) comprises a plurality of liquid retarding layers. Said barrier (5) may be in the form of a patch or discrete layer(s) and from about 50% to about 75% of a perimeter thereof is joined to said insert (4) and/or panels (2,3) such that an un-joined portion, or non-joined portion or unattached portion, of said perimeter forms an opening to a containment pocket.

As used herein the term "patch" and "discrete layer" are preferably synonymous and may be interchangeable as they may both refer to an element forming the transversal waist barrier(s).

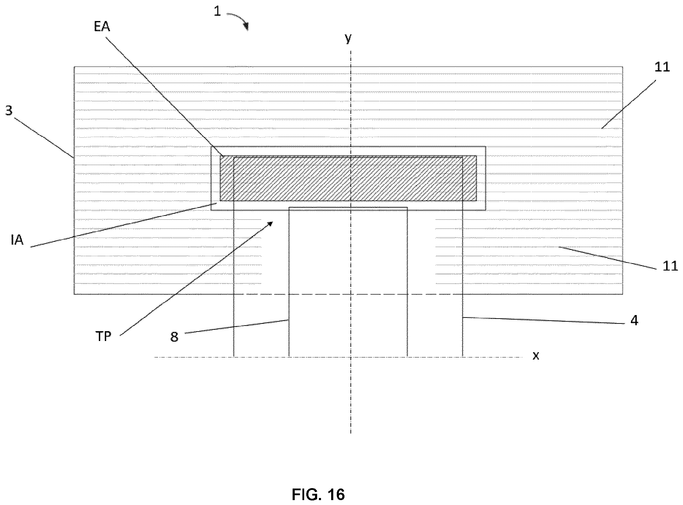

As for example further illustrated in Fig.16, the front and/or back panels (2, 3) preferably comprise a plurality of elastic strands, preferably sandwiched between two or more nonwoven layers as typically described herein, and wherein said front and/or back panels (2, 3) may comprise a tummy pause (TP) being free of said elastic strands or comprising elastic strands that have been rendered inelastic by deactivation (e.g. by deactivation/severing of the elastics via a knife or similar process); and wherein the barrier (5) overlaps said tummy pause (TP), preferably overlaps the entire width of said tummy pause (TP) along an axis parallel to the transversal centerline (x). Preferably, the tummy pause (TP) has a width extending along an axis parallel to the transversal centerline (x), and said width being more than 60%, preferably more than 65%, more preferably from 70% to 180%, even more preferably from 75% to 150%, of a total width of the absorbent insert (4). Preferably, at least a portion of the barrier (5) is joined to the front and/or back panels (2, 3) at the tummy pause (TP), more preferably wherein a portion of the perimeter of the patch (18) being opposite the opening to the containment pocket is joined to the front and/or back panels (2, 3) at the tummy pause (TP). Advantageously, this may allow to reduce wrinkle formation on the waist belts of the article as well as the formation of non-uniform wrinkles on the barrier itself. Moreover, this arrangement may aid to bring at least partial elasticisation to the tummy pause area for a tighter fit whilst limiting core deformation or compromising core integrity upon wetting and swelling of the absorbent core comprised by the insert.

Preferably, the plurality of liquid retarding layers, the patch and/or discrete layer, comprise at least two, preferably more than two, layers selected from the group consisting of hydrophobic nonwovens, films, and/or strands, more preferably a laminate comprising a film or a plurality of strands, preferably the film or strands being elastic, sandwiched between two of said hydrophobic nonwovens. Advantageously this generally allows for improved exudate leakage protection.

According to a further embodiment, each hydrophobic nonwoven is a multi-layer nonwoven comprising at least two, preferably at least three, layers selected from a spunbond nonwoven and a meltblown nonwoven, more preferably said nonwoven being selected from a spunbond-meltblown (SM) nonwoven, a spunbond-meltblown-spunbond (SMS) nonwoven, and a spunbond-meltblown-meltblown-spunbond (SMMS) nonwoven. Advantageously the presence of meltblown layers in the nonwoven allows for better barrier effects arising from its finer fiber structure and smaller openings between fibers (compared e.g. to spunbond layers) and yet retain sufficient mechanical strength for resistance to forces that are applied upon movement of the subject especially in such gasketing locations in direct/tight contact with the skin.

According to a further embodiment, the hydrophobic nonwovens comprise, preferably consist of, synthetic fibers; preferably wherein said fibers comprise a material selected from polypropylene or derivatives thereof, polyethylene or derivatives thereof, polyester or derivatives thereof, polylactic acid or derivatives thereof, polylactic-co-glycolic acid or derivatives thereof, polyhydroxyalkanoates or derivatives thereof, and mixtures thereof.

For example, the patch can be in the form of a laminate of three layers, where a layer consisting of elastic strands is sandwiched between two SMS nonwovens layers.

Preferably the patch or discrete layer (18) has a basis weight of less than 50 g/m$^2$, preferably from 8 g/m$^2$ to 45 g/m$^2$. The patch may be elastic, or may comprise elastic material such as one or more elastic strands and/or elastic foam, and may comprise micro-openings sized to allow at least some water vapour to permeate therethrough. Preferably the patch has a ratio of the MD load at break to the CD load at break of less than about 8, and a machine-direction notched trapezoidal tear strength of at least about 25 g. The patch may further have an opacity of at least about 50%. Preferably wherein said patch is substantially void of titanium dioxide; and/or wherein the patch comprises an olefin block copolymer which is ethylene-based or propylene-based or combinations thereof; and/or wherein said patch comprises from about 30% to about 60% by weight of a filler; and/or wherein the patch is a coextruded multilayer film. Advantageously this allows for identification of said barrier without the use of significant amounts of pigment.

According to a further embodiment, the opening is positioned closer to a transversal centerline (x) than the joined portions of the perimeter of the patch.

According to an embodiment, the joined portion of the perimeter of the patch or discrete layer is joined by adhesive and/or mechanical bonding having a shape and/or pattern that is substantially C-shaped or U-Shaped, preferably such that at least one of the longest sides and at least two opposite portions of the shortest sides of said patch are joined and wherein at least one of the longest sides of said patch is un-joined or unattached to the absorbent insert (4). Given that only a portion of the patch is joined to the absorbent insert, i.e. the joined portion, hence there is a portion of the patch that is unattached or un-joined, i.e. the un-joined or unattached portion.

Preferably, at least a portion of the perimeter of the patch (18), preferably comprising at least a portion or the majority of the two shortest sides, is bonded to a pair of oppositely disposed cuffs, most preferably wherein the said patch (18) is joined to either a garment facing surface of said cuffs or a body facing surface of said cuffs. The cuffs may be joined to the topsheet of the insert (4) and generally serve the purpose of limiting lateral leakage. An advantage of joining the patch to the garment facing surface of the cuffs is that further leakage prevention is ensured since any internal leakage from the barrier is further retarded by the cuff material itself compared to when the patch is applied on a body-facing side/surface of the cuff. Such arrangement may be achieved by for example applying the cuffs after the step of joining the patch to the insert at a process downstream position. An advantage of joining the patch to the body-facing surface of the cuffs is that the tension and up-standing effect of the cuff aids to push-up the transversal barrier formed by the patch and hence maximizing its height extension and tight gasketing fit to the skin upon use, whilst avoiding process complexities in the assembly of such products. In either scenario, it remains desirable to join at least a portion of the said patch/barrier to said cuffs.

For example, the patch or discrete layer (18) is substantially rectangular with 2 longest sides oppositely disposed and connected by 2 oppositely disposed shortest sides. The longest sides extending in the transversal direction and the shortest sides extending in the longitudinal direction. The un-joined or unattached side is that closest to the centerline x and furthest to the transversal edge of the front and/or back panel and preferably corresponds to one of the longest side, meaning the longest side more proximal to the centerline. The present disclosure is not limited to rectangular shapes. As long as the patch or discrete layer 18 can cover a portion of the absorbent insert proximal to the transversal edge and serve as a containment pocket, the patch can be substantially triangular, pentagonal, elliptical, or any polygonal shape.

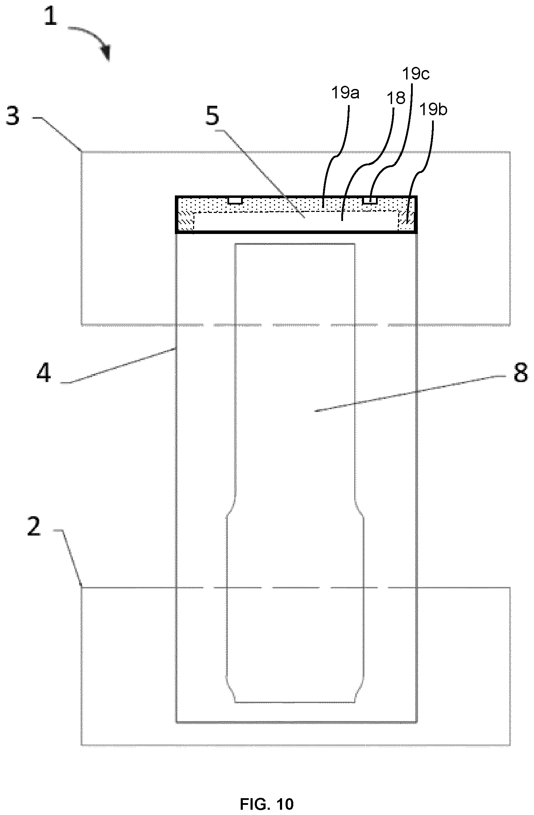

Said patch or discrete layer (18) preferably comprising an elastic film or strands is preferably in the form of a rectangular patch. Said rectangular patch extends along the transversal edge of the absorbent insert (4) and partially along the longitudinal edges of the absorbent insert (4) as illustrated in FIG. 10. In other words, said patch (18) covers the portion of the absorbent insert (4) proximal to the front and/or back transversal edge, preferably the portion of the absorbent insert (4) proximal to the back transversal edge.

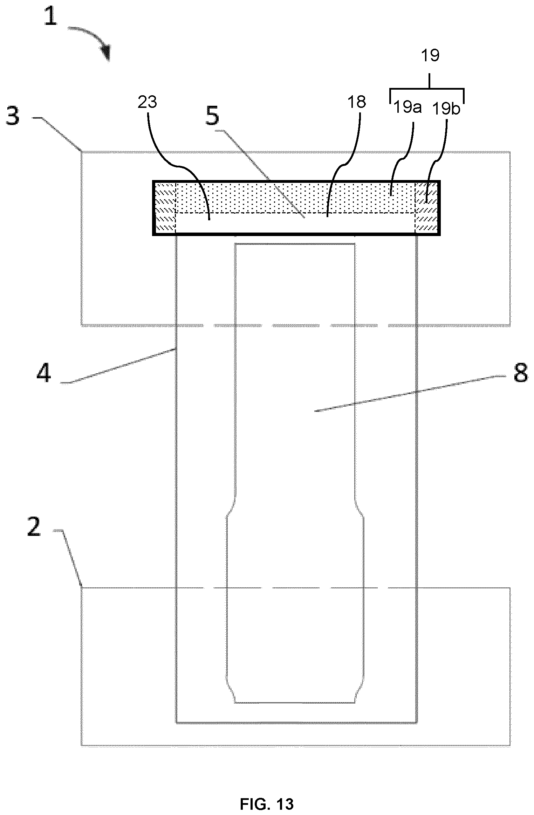

The discrete layer or patch (18) in embodiments herein may be bonded, meaning joined or attached or associated, to the absorbent insert (4) by at least one bonding means (19). Bonding means comprise and are not limited to for example adhesive bonding and/or mechanical bonding and/or ultrasonic bonding. The discrete layer or patch (18) can be joined to the absorbent insert (4) by two or more bonding means. As illustrated in FIG. 10, the bonding means comprises here adhesive bonding (19a) in an area arranged along the transversal edge of the absorbent insert (4) and mechanical bonding (19b) in an area arranged along the longitudinal edge of the portion of the absorbent insert (4) proximal to the front and/or back transversal edge, preferably the back transversal edge. The bonding means comprise only adhesive bonding (19a) arranged along the transversal and longitudinal edges of the portion of the absorbent insert (4) proximal to the front and/or back transversal edge. Arranging the bonding means at the transversal and longitudinal edges of the absorbent insert (4) enables to have a proper waist transversal barrier (5) and contain any exudate within the absorbent insert (4). Preferably, the opening formed by the unattached, or un-joined portion, is positioned closer, or at equal distance for the edges of the shortest sides, to a transversal centerline (x) than the joined portions of the perimeter of the patch. As illustrated in FIG. 13, the bonded portion is represented by reference (19) and the unattached portion is represented by reference (23).

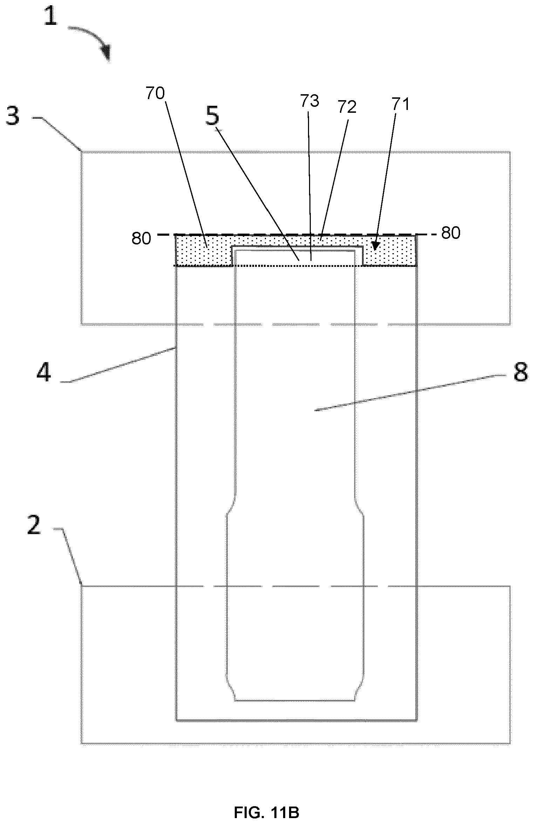

When looking at the absorbent article laid flat on a surface from above, the unattached portion (23) generally covers a portion of the absorbent insert (4), namely the portion proximal to the transversal edge of the absorbent insert (4). According to some embodiments, the unattached portion (23) can optionally further cover a portion of the absorbent core (8) as illustrated in FIG. 11B. The bonded or joined portion 19 can either cover a portion of the absorbent insert (4), namely the portion proximal to the transversal edge of the absorbent insert (4) only (FIG. 10) or cover a portion of the absorbent insert (4), namely the portion proximal to the transversal edge of the absorbent insert (4) and a portion of the back panel (3) (FIG. 13) depending on how the patch 18 is arranged on the absorbent article 1. The patch or discrete layer 18 can be joined to the outer panel (3), namely to the inner nonwoven layer (9), and/or the patch or discrete layer 18 can be joined to absorbent insert (4).

According to an embodiment, said barrier (5) comprises one or more of an elastic film and an elastic strand, and wherein the elastic film and/or strand(s) form an elastic area (EA) and wherein the elastic area is less than a patch area such that a substantially inelastic area (IA) is positioned outboard of the elastic area, preferably wherein the inelastic area comprises at least one of: an area free of elastic film and/or strand(s); and an area wherein the elastic film and/or strand(s) has been de-activated. Deactivation of elastic includes cutting or severing elastic for example using a knife roller combined to an anvil roller. Other methods of deactivation include and are not limited to melting, using heat and/or chemical agents, or any other means of providing stiffness e.g. glue in non-stretched state or addition of other stiffeners.

According to an embodiment, at least a portion of the substantially inelastic area (IA) is joined to the absorbent insert (4) and/or panel(s) (2, 3). Advantageously this allows for optimal anchoring.

According to an embodiment, the un-joined portion of said perimeter comprises a further elastic material, preferably in the form of one or more elastic strands such as LYCRA®.

According to an embodiment, the elastic area (EA) comprises a plurality of wrinkles; and/or wherein the elastic area comprises a plurality of apertures. According to this embodiment said least one nonwoven layer and elastic film and/or strands are joined by mechanical bonding, at a plurality of discrete bonding elements and wherein each said patch comprises a plurality of wrinkles having peaks and troughs formed on at least one of said nonwoven layer wherein said patch comprises an average of no more than two, preferably no more than one, wrinkles between two consecutive discrete bonding elements along a patch length extending substantially parallel to the transverse axis. The patch (18) comprises a wrinkle distribution of at least 1.1 wrinkles/mm, according to the method herein.

The apertures are preferably formed at bonding sites within a bonding pattern formed by mechanical bonding for example. Preferably, the openings are made by laser perforation or mechanical piercing such as by one or more needles and/or pins, and preferably wherein the discrete bonding elements are made by ultrasonic bonding preferably such that the nonwoven web(s) and film are melt-fused such to form an aperture at a center of each said discrete bonding elements, preferably wherein the openings are made by laser perforation. In an embodiment, the manufacturing process herein may be configured to aperture the assembled laminate after assembly for example by inserting pins or needles through the laminate (preferably in combination with heat e.g. the pins or needles are heated for piercing the laminate). However, it has been observed that when inserting pins through the laminate, the nonwovens may be deformed, and protrusions may be created where the apertures are formed. Such protrusions may extend outward from one surface of the laminate. As such, the surface of the resulting laminate that includes the protrusions protruding therefrom may feel relatively rough and thus can detract from other desirable features of the elastic laminate, such as softness.

According to an alternative or additional embodiment, the at least portion of said absorbent insert (4) proximal to the front and/or back transversal edges thereof comprises one or more folds forming the transversal waist barrier(s) (5), preferably wherein said fold is substantially U-shaped or C-shaped. Advantageously such fold of the absorbent insert allows to form a liquid impermeable barrier to exudates, and particularly the U-shape or C-Shape (vs other shapes) allows to form a maximised pocket for exudate collection with the minimum use of material.

According to an embodiment, the folded portion of said absorbent insert (4) is joined to the body-facing surface of the topsheet (6) at a pair of oppositely disposed extremities of the absorbent insert (4) along a portion of the left and right longitudinal edges thereof, generally wherein said oppositely disposed extremities are connected by a fold of said folded portion of said absorbent insert (4), such that a pocket is formed that acts as a barrier to exudates in both the longitudinal direction (i.e. substantially parallel to the longitudinal axis y) and the transverse direction (i.e. substantially parallel to the transverse axis x). The said extremities of the absorbent insert (4) may be joined by adhesive and/or mechanical bonding such as ultrasonic bonding, thermal bonding, and/or pressure bonding and the like.

In an embodiment, the absorbent insert (4) comprises a substantially liquid permeable topsheet (6), a substantially liquid impermeable backsheet (7), and an absorbent core (8) sandwiched therebetween, preferably wherein the absorbent core (8) is positioned inboard of the perimeter of said absorbent insert (4) when viewed in a planar direction (as generally depicted and illustrated in the figures herein and that typically corresponds to a plane formed by the x and y axis described and illustrated herein) such that the one or more folds are substantially free of absorbent material. Advantageously, a fold that is substantially free of absorbent material allows for a more flexible fold that can allow said barrier to better stand upright upon application of tension and yet limit piercing of the topsheet and/or backsheet that could arise if exerting a folding force in an area of the absorbent insert comprising SAP particles.

According to an embodiment the transversal barrier (5), meaning with a patch or by folding the absorbent insert, is breathable and has a water vapor transmission rate (WVTR) of less than about 2300 $g/m^2x24hr$ at an elongation of about 0% and a water vapor transmission rate (WVTR) of more than about 2800 $g/m^2x24hr$ at an elongation of about 100%, according to the test method herein; preferably a water vapor transmission rate (WVTR) of less than about 2250 $g/m^2x24hr$, preferably less than about 2150 $g/m^2x24hr$, even more preferably from about 500 $g/m^2x24hr$ to about 2000 $g/m^2x24hr$, at an elongation of about 0%; and a water vapor transmission rate (WVTR) of more than about 2850 $g/m^2x24hr$, preferably more than about 2950 $g/m^2x24hr$, more preferably more than about 3000 $g/m^2x24hr$, even more preferably from about 3100 $g/m^2x24hr$ to about 5500 $g/m^2x24hr$, at an elongation of about 100%, as measured according to the test method herein. Advantageously this provides for modular breathability that is activatable as the material stretches. This modular breathability accommodates for larger subjects that may need more enhanced breathability than smaller subjects.

The inner and outer nonwoven layers (9, 10) may be the same or different meaning that they may have the same or different properties selected from basis weight (in gsm) and composition. In an embodiment, both the inner and outer nonwoven layers (9, 10) each comprise a spunbond nonwoven, typically comprising monocomponent fibers of polypropylene, and each have a basis weight of from 10 $g/m^2$ (gsm) to 30 $g/m^2$ (gsm), preferably from 12 $g/m^2$ (gsm) to 25 $g/m^2$. The outer nonwoven layer (10) may have a basis weight that is higher than the inner nonwoven layer (9). Advantageously, although it is customary to have the higher basis weight nonwoven on the inner nonwoven wearer skin facing side, in barrier embodiments herein (especially when the outer nonwoven layer is folded and/or extends over the absorbent insert; and/or when the transversal barrier is elasticised) such arrangement provides for added mechanical integrity desirable for resistance to tear on elastification and/or added softness for the wearer on the front and/or back waist regions up to said barrier location.

Preferably, and as exemplified in Fig. 1, the elastic material (11), such as the plurality of elastic strands, is/are deactivated in an area of overlap of the absorbent insert (4) when viewed in a planar direction. The deactivation is preferably achieved by cutting of the elastic strands such as to render the deactivated region of the panel(s) (2, 3) substantially inelastic and regions neighbouring said deactivated region and comprising said elastic strands being elastic. Typically the absorbent core (8) of the absorbent insert (4) is positioned within the deactivated region such that it substantially does not overlap the elastic strands (11). Advantageously this allows for improved core integrity as risks of core collapse due to excessive tension from the stretched elastics when the article is worn is limited.

Preferably more than 55%, preferably more than 60%, even more preferably from 65% to 95%, of the total surface area (generally when viewed in a planar direction) of the transversal waist barrier(s) (5) is positioned to overlap the deactivated region of the panel(s) (2, 3). Advantageously this allows for improved fit of said barrier when the article is worn by a subject.

In addition or alternatively, more than 50%, preferably more than 60%, even more preferably from 65% to 95%, of a total transversal length (generally substantially parallel to the transversal axis x) of the transversal waist barrier(s) (5) overlaps the deactivated region of the panel(s) (2, 3). Especially when the transversal waist barrier(s) is elastic, this arrangement may advantageously not only improve core integrity but further avoid excessive tension build-up.

In an embodiment, the transversal waist barrier(s) (5) is elastic, preferably comprising an elastic material selected from the group consisting of an elastic film and one or more elastic strands. Advantageously this allows for application of tension that permits said barrier to stand upright in close contact to the skin when the article is worn and under stretch.

The elastic strands in any of the embodiments herein may be strand-coated with adhesive prior to joining to one or more layers such as the inner and outer nonwoven layers (9, 10) to form elastic panels (2, 3) and/or the backsheet (7), outer cover (7'), and/or outer nonwoven layer (10) to form the elastic transversal waist barrier(s) (5). Advantageously this allows to join the one or more layers together by the said strand-coated elastic strands and by doing so limit the amount of adhesive used with resulting improved softness as well as reduced waste. Alternatively, or additionally, adhesive in the form of a plurality of stripes is applied such as by slot coating.

In a preferred embodiment, the one or more elastic strands of the transversal waist barrier(s) (5) comprise at least one flat elastic (such as elastics having a cross-section with an aspect ratio of longest to smallest dimension of greater than 1, preferably greater than 1.5, even more preferably greater than 2), preferably in combination with at least one or at least two round elastics (such as elastics having a cross-section with an aspect ratio of longest to smallest dimension of about 1). Although flat elastics are generally more expensive, they are advantageous in providing better gasketing effects and comfort on the wearer's skin, by combining flat and normal/round elastics one can achieve the desired gasketing and comfort whist limiting the cost.

When one or more, preferably only one, flat elastic is used it is preferably positioned adjacent to an apex of the transversal waist barrier(s) (5) that may be closest to a transversal edge of the absorbent insert (4) and further round elastic(s) positioned further from said apex compared to the flat elastic(s). Advantageously this allows for the flat elastic to be positioned at the closest contact position to the wearer with other elastics being inboard (or further away) therefrom hence contributing to the desired tensile strength yet permitting to lower the overall density/dtex of the flat elastic and hence also cost, with limited impact on performance.

When elastic strands are used, they may have a dtex in the range of from 350 to 1100, preferably from 400 to 1000, even more preferably from 450 to 900. When elastic strands are used in all of the front and/or back panels (2, 3) and the transversal waist barrier(s) (5), the elastic strands of said panel(s) (2, 3) have a dtex that is equal to or greater than, preferably greater than, that of the elastic strand(s) of said transversal waist barrier(s) (5). Preferably the elastic strand(s) of said transversal waist barrier(s) (5) have a dtex that is from 40% to 85%, preferably from 45% to 75%, even more preferably from 50% to 70%, of the dtex of the elastic strands of said panel(s) (2, 3). Advantageously, this allows said barrier(s) to stand up and provide a gasketing effect close to the skin of the wearer without flattening or adding excessive resistance to stretch that would otherwise impact fit and comfort. The tension differential that results entails that a lower tension in said barrier(s) compared to the belt(s) is generated to a degree that upon stretching of the belt(s) said barrier(s) stand-up away from said belt(s).

The tensile stress (N/m) of the entirety of the front and back panels (2, 3), respectively, may be profiled in order to provide the functional benefits of the present disclosure, such as ease of stretch and application, while also maintaining certain force during wear, to prevent the article from sagging after loading. When the elasticity of the front and back panels (2, 3) are provided by a plurality of elastic strands (11) running in the transverse direction, the tensile stress may be adjusted by one or more of the following methods; 1) elongation rate of the elastic strands (11); 2) density (dtex) of the elastic strands (11); 3) longitudinal pitch of multiple elastic strands (11); and 4) effective length of elasticity of the elastic strands (11) in the transverse direction. By elongation, "0% elongation" is meant the original length of the elastic member.

The front and back panels (2, 3) may each be divided into multiple zones spanning in the transverse direction and defined by its location from the distal edge to the proximal edge relative to the percentage of the seam length wherein the distal edge is considered 0% and the proximal edge is considered 100%. The multiple zones may be configured to provide different tensile stress, or different functions to the front and back panels (2, 3), respectively.

In an embodiment, one or more second elastic strands (12) are comprised directly or indirectly between the outer nonwoven layer (10) and the backsheet (7), preferably between a body-facing surface of the outer nonwoven layer (10) and a garment-facing surface of the backsheet (7). An example of an indirect elastic arrangement is the presence of an outer cover layer (7') as described herein (that would generally be interposed between the backsheet (7) and the one or more second elastic strands (12)), and an example of a direct elastic arrangement is the absence of an outer cover layer (7') as described herein. Advantageously this arrangement allows for a simple yet effective elastification of said barrier(s) permitting optimal gasketing effects.

Preferably, the one or more second elastic strands (12) extends along the entire transversal length of the transversal waist barrier(s) (5) and beyond, preferably crossing the imaginary longitudinal axis (y) in a direction substantially parallel to the imaginary transverse axis (x). Preferably the one or more second elastic strands (12) extends from a first position substantially adjacent a seam edge of the outer nonwoven layer (10) to a second position substantially adjacent an opposite seam edge of the outer nonwoven layer (10) with the imaginary longitudinal axis (y) being positioned therebetween, more preferably wherein the one or more second elastic strands (12) extends from said first position to said second position and crosses and/or overlaps the entire transversal waist barrier(s) (5) generally along the imaginary transverse axis (x). Advantageously this allows not only to form a standing barrier upon stretching but further aids better fit on the wearer's front and/or back waist.

In an embodiment, the absorbent core (8) comprises absorbent material selected from the group consisting of superabsorbent polymer particles and/or fibers; and cellulose fibers, preferably wherein said absorbent material is enclosed within a core wrap comprising top and bottom core wrap layers being a same nonwoven layer folded to sandwich the absorbent material therein or distinct nonwoven layers joined together to sandwich the absorbent material therein. The superabsorbent polymer particles and/or fibers may be comprised in an amount of greater than 50%wt, preferably greater than 60%wt, even more preferably greater than 70%wt, most preferably from 75% to 100%wt, by total weight of absorbent material.

In an embodiment, the back panel (3) is wider (generally in a direction substantially parallel to the longitudinal axis y) than the front panel (2) such that a length of the seams is substantially equal to a width (generally in a direction substantially parallel to the longitudinal axis y) of the front panel (2) and less than a width of the back panel (3); or wherein the front panel (2) is wider than the back panel (3) such that a length of the seams is substantially equal to a width of the back panel (3) and less than a width of the front panel (2). Advantageously this may permit comfort as well as improved protection compared to symmetric arrangements.

It is preferred that the backsheet (7) comprises a further outer cover layer (7') on a garment-facing surface thereof, preferably wherein said outer cover layer (7') comprising one or more nonwoven layers, and/or wherein the backsheet (7) comprises a further outer cover layer (7') that is shorter in the longitudinal direction (generally parallel to the longitudinal axis y) than the topsheet (6) and/or backsheet (7) such that at least a portion of an overlap length between the absorbent insert (4) and the panel (2, 3) is free of said outer cover layer (7') and preferably wherein the backsheet (7) is directly joined to, or is in contact with, the panel (2, 3) in said portion of an overlap length between the absorbent insert (4) and the panel (2, 3) free of said outer cover layer (7'). Advantageously this allows for improved softness in areas where needed and yet allow for material savings thus permitting both reduced cost and waste.

The outer cover layer (7') is preferably different from the inner and/or outer nonwoven layers (9, 10) meaning that they may have different properties selected from basis weight (in gsm) and composition. In an embodiment, the outer cover (7') comprises a spunbond nonwoven preferably comprising bicomponent fibers typically selected from the group consisting of polypropylene and polyethylene. The outer cover (7') preferably has a basis weight that is less than the basis weight of the inner and/or outer nonwoven layers (9, 10). The outer cover (7') may have a basis weight of from 8 $g/m^2$ (gsm) to 20 $g/m^2$ (gsm), preferably from 10 $g/m^2$ (gsm) to 15 $g/m^2$. Advantageously, the outer cover provides for softness to the touch to the care giver yet, as selected, it limits stress build-up particularly in embodiments with reduced length in the longitudinal direction as described above. Indeed without wishing to be bound by theory, stress build-up in the transition region corresponding to the terminal edge of the outer cover may result in a higher risk of tearing in the absorbent insert to panel joint.

In a preferred embodiment, the absorbent insert (4) is folded-over to form the waist barrier(s) (5) such that at least a portion of the topsheet (6) of the non-folded part of said insert (4) may come into contact with the folded part of said insert (4). Advantageously this allows said barrier to overlap the topsheet so as to help guide the exudates therethrough and into the core once stopped by said barrier.

Preferably, the waist barrier(s) (5) extends from 5% to 50%, preferably from 10% to 40%, even more preferably from 15% to 30%, of the total folded length (TFL) of the outer nonwoven layer (10) along the longitudinal axis (y). Advantageously, this may allow formation of a sufficiently large pocket yet avoid excessive resizing of the absorbent insert that may add cost and waste.

In an embodiment, the substantially liquid impermeable film layer or backsheet (7) comprises indicia, preferably a coloured print, at a position corresponding to the transversal waist barrier (5); and/or wherein the one or more second elastic strands (12) are coloured; and/or wherein a garment facing surface of the outer nonwoven layer (10) is coloured; and/or wherein the transversal waist barrier (5) comprises indicia selected from a coloured print and coloured adhesive (preferably comprising one or more pigments). Advantageously this allows the user or care giver to better visualise said barrier(s) so as to aid correct positioning and placement thereof onto a subject.

In an embodiment, the absorbent article further comprising a pair of longitudinally extending cuffs positioned along left and right longitudinal edges of the absorbent insert (4), and wherein the transversal waist barrier(s) (5) is positioned above said cuffs such that said barrier(s) (5) is closer to a wearer's skin than said cuffs; preferably wherein the cuffs are joined to a body-facing surface of the topsheet (6) and wherein each of said cuffs is folded and joined to itself on a body-facing surface thereof at a joining position (JP) corresponding to the transversal waist barrier(s) (5), preferably said joints or joining positions (JP) extending along a lengthwise portion of, and being substantially adjacent to, the left and/or right longitudinal edges of the absorbent insert (4). Advantageously this reduces risk of leakage multidirectionally and prevents leakage to seep through between the transversal barrier and cuffs unlike when otherwise arranged.

Preferably the transversal waist barrier (5) is an up-standing barrier generally arranged to block substantially liquid exudates from flowing therethrough. Preferably, the liquid impermeable film layer is breathable and has a basis weight of less than 50 g/m$^2$, preferably from 8 g/m$^2$ to 45 g/m$^2$. The film may be elastic (or may comprise elastic material such as one or more elastic strands and/or elastic foam) and may comprise micro-openings sized to allow at least some water vapour to permeate therethrough. Preferably the film having a ratio of the MD load at break to the CD load at break of less than about 8, and a machine-direction notched trapezoidal tear strength of at least about 25 g.

When the liquid impermeable film layer is a discrete layer and generally applied in the form of a patch joined to said insert via suitable bonding techniques such as adhesive and/or mechanical bonding as described in more detail herein, it is preferably elastic. "Elastic," "elastomeric," and "elasticized/elasticised" herein generally mean the ability of a material to stretch by at least 100% without rupture or breakage at a given load, and upon release of the load the elastic material or component exhibits at least 70% recovery (i.e., has less than 30% set). In this embodiment, it is preferable that the liquid impermeable film layer is joined to the absorbent insert by a combination of adhesive and mechanical bonding.

The adhesive may be applied in a first joining zone or bonding zone and the mechanical bonding may be applied on a second joining zone, and preferably wherein the adhesive is continuously applied along the first joining zone at an area generally comprising substantially the entire transversal-waist-barrier-width (typically extending along an axis parallel to the transversal axis x, and generally adjacent the front and back transversal edges). The first and second joining zones may be proximal to each other (or adjacent) such that the majority, preferably substantially all, the area of adhesive is not overlapped by mechanical bonds. Advantageously this allows for extra anchoring and breathability generally afforded by the mechanical bonding yet maintain an appropriate liquid seal to limit leakage through said barrier along the transversal edge of the absorbent insert.

The impermeable film layer, when present, may be discrete and joined to the absorbent insert (4) by at least one of adhesive and mechanical bonding such as ultrasonic bonding, heat bonding, pressure bonding, and the like; or may be an integral part of the absorbent insert (4) and may form barriers herein such as by folding a portion thereof as described in more detail hereinbelow.

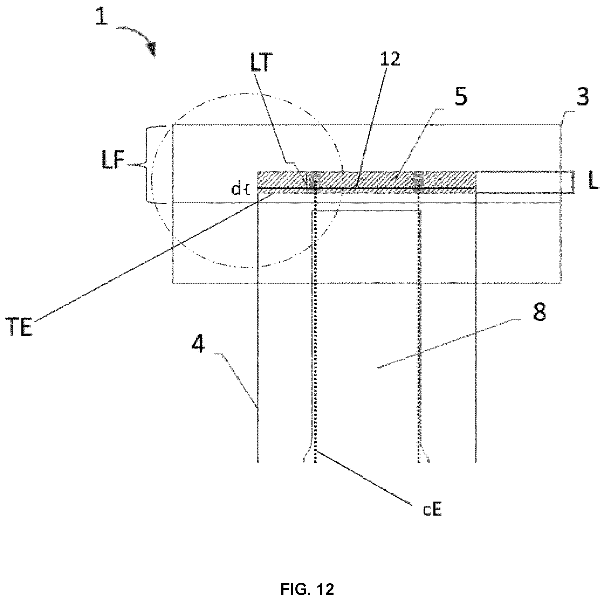

As shown in FIG. 12, in this embodiment, a portion of the absorbent insert proximal (typically adjacent) to the front and/or back transversal edges thereof may be folded onto itself and the fold (as described in embodiments herein) has a length (L) in a longitudinal direction running substantially parallel to a longitudinal axis (y), and wherein said insert (4) further comprises a pair of longitudinally extending cuffs positioned along left and right longitudinal edges thereof and joined thereto along a tack-down length (LT) extending from a position proximal (preferably adjacent) to at least the back transversal edge to a tack-down terminal position longitudinally displaced therefrom along said longitudinal axis (y), and wherein the tack-down length (LT) is less than or equal to about 2L, preferably said cuffs being joined to a body-facing surface of the topsheet (6). It is understood herein that whilst this embodiment makes specific reference to the "back" transversal edge, when the transversal waist barrier(s) (5) are positioned at the front of the article, the same dimensional characteristics apply with respect to the "front" transverse edge. Advantageously, this allows for a reduced tack-down length that permits to maximise the volume of the containment pocket formed by extending the lateral cavities when the fold stands up in contact with the user when the article is worn.

The back and/or front transversal edge (generally in folded state, as can be generally seen in the figures) typically coincides with a terminal edge (TE) of the transversal waist barrier (5) forming a pocket opening.

Preferably, the transversal waist barrier (5) comprises one or more second elastic strands (12) positioned at a distance (d) from a terminal edge (TE) of the transversal waist barrier (5) forming a pocket opening, and wherein the distance between the elastic closest to said terminal edge is less than about 0.6L, preferably from 0.15L to 0.5L, even more preferably from 0.20 to 0.45L. Advantageously this permits the transversal barrier to more easily stand-up and make a seal with the wearer's skin and preferably yet be sufficiently displaced from the terminal edge to minimize risk of elastic exposure to the skin of a wearer.

Preferably, the font and/or back panels (2, 3) are elastic and comprise an inner nonwoven layer (9), an outer nonwoven layer (10), and an elastic material (11) sandwiched therebetween, wherein the elastic material is selected from the group consisting of an elastic film and a plurality of elastic strands. Preferably, the outer nonwoven layer (10) is wider than the inner nonwoven layer (9) (generally in a direction substantially parallel to the longitudinal axis y) and is folded over said inner nonwoven layer (9) such that it overlaps the portion of said absorbent insert (4) proximal to the front and/or back transversal edges and generally over a fold length (LF), preferably such that said outer nonwoven layer (10) overlaps both a portion of the backsheet (7) and a portion of the topsheet (6) that remains exposed from said one or more transversal waist barrier(s) (5) such to form a flange ($F_L$). Advantageously this allows to cover the backsheet with nonwoven so that the backsheet does not come into contact with the skin of a wearer which could otherwise result in skin irritations and/or less comfort.

The absorbent insert (4) may comprise a topsheet selected from an embossed nonwoven and/or a carded air-through-bonded nonwoven. The embossed nonwoven may comprise a spunbond nonwoven. Advantageously the topographical surface of such particular nonwoven selection allows for the formation of peaks and slumps that acts to not only slow down the movement of liquid stool towards the transversal edges but further allows for containment ridges in areas overlapping said barrier(s) (5).

Preferably, the thickness of the absorbent insert (4) in an overlap area of said waist barrier (5) (e.g. an area of the absorbent insert (4) overlapping with the waist barrier (5)) is less than the remaining thickness of said insert (4) (e.g. in areas other than the overlap area); and/or wherein the amount of absorbent material, if and/or when present, in in an overlap area of said waist barrier (5) is less (preferably at least 5%wt less, preferably at least 10%wt less, even more preferably at least 15%wt less, even more preferably at least 20%wt less) than the amount of absorbent material in other areas of said insert. Generally said overlap area further overlapping with the front and/or back panels respectively. Advantageously this allows for a larger pocket for exudate collection that synergistically cooperates with the overlapping front and/or back belts.

Preferably, a method for the manufacture of an absorbent article as described herein, comprises the steps of:

- providing a front panel comprising an outer nonwoven and an inner nonwoven, for example a front outer nonwoven and a front inner nonwoven
- providing a back panel comprising a back outer nonwoven and a back inner nonwoven, for example a back outer nonwoven and a back inner nonwoven;
- providing an absorbent insert comprising front and back transversal edges and left and right longitudinal edges connecting the front and back transversal edges to form a perimeter thereof;
- joining said absorbent insert to said front and back panels, namely to the inner nonwovens, namely to the front and back inner nonwoven,
- folding both outer nonwoven and inner nonwoven of the front panel over a portion of the front transversal edge of the absorbent insert and/or folding both outer nonwoven and inner nonwoven of the back panel over a portion of the back transversal edge of the absorbent insert (4), namely folding front outer nonwoven and front inner nonwoven over a portion of the front transversal edge of the absorbent insert and/or folding back outer nonwoven and back inner nonwoven over a portion of the back transversal edge of the absorbent insert (4),
- joining said front and back panels (2, 3) together along a pair of oppositely disposed side seams.

The above step are preferably in the above described sequential order.

The method preferably comprises further additional steps selected from:

- applying a first patch of adhesive (A1) onto the side of the outer nonwoven (NW2) facing the inner nonwoven (NW1); and/or
- folding a portion of the outer and inner nonwovens (NW1,NW2) onto the inner nonwoven (NW1); and/or
- applying a second patch of adhesive (A2) onto the side of the inner nonwoven (NW1) facing away from the outer nonwoven (NW2); and/or
- folding a portion of the outer and inner nonwovens (NW1,NW2) onto the inner nonwoven (NW1) over the second patch of adhesive (A2) and/or
- folding the outer and inner nonwovens (NW1,NW2) so that the first elastic region (ER1) and the second elastic region (ER2) are in facing relationship, at least partially.

In an embodiment, a method for the manufacture of an absorbent article (1) may comprise the steps of:

- providing a front panel (2);
- providing a back panel (3);
- providing an absorbent insert (4) comprising front and back transversal edges and left and right longitudinal edges connecting the front and back transversal edges to form a perimeter thereof;
- providing a discrete layer or patch (18) to cover at least a region of the absorbent insert proximal to the front and/or back transversal edges to form transversal waist barrier(s) (5) ; and at least one of:

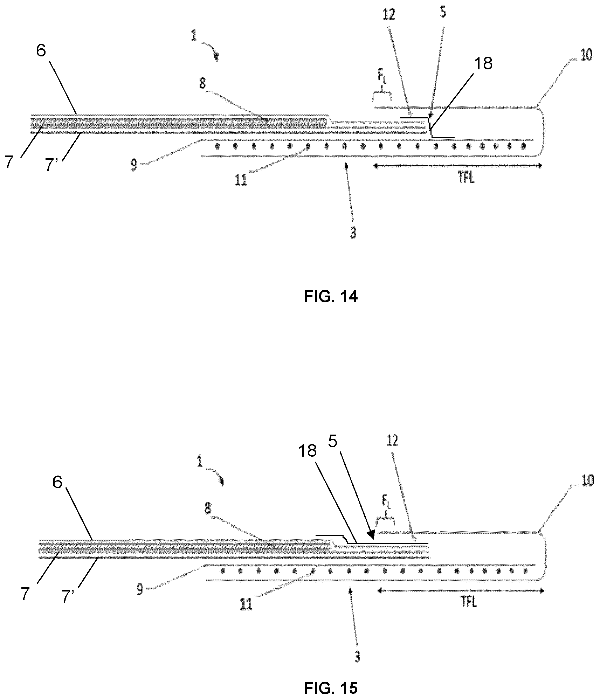

  • joining said absorbent insert (4) to said front and back panels (2, 3) and joining said patch (18) to said front or back panels (2, 3) to cover at least a region of the absorbent insert (4) proximal to the front and/or back transversal edges as illustrated in FIG. 14;

- joining said patch (18) to absorbent insert (4) by bonding means to cover at least a region of the absorbent insert proximal to the front and/or back transversal edges and joining said insert (4) to said front and back panels (2, 3) as illustrated in FIG. 15; and/or

- joining said insert (4) to said front and back panels (2, 3) and joining said patch (18) to absorbent insert (4) by bonding means to cover at least a region of the absorbent insert proximal to the front and/or back transversal edges as illustrated in FIG. 15; and

- further joining said front and back panels (2, 3) together along a pair of oppositely disposed side seams.

These steps may be sequential in the order listed here-above or may be sequential but with the steps of joining said patch and joining said inserts being carried out substantially concurrently.

According to an embodiment, the bonding means comprise applying a pattern of adhesive onto a discrete layer or patch (18) and/or applying a pattern of adhesive onto a portion of the absorbent insert (4) proximal to the front and/or back transversal edge said pattern of adhesive extending all along the transversal edges and partially along the longitudinal edges of the patch and/or absorbent insert (4) and applying said patch or discrete layer onto the top surface of the absorbent insert (4) at a portion of the absorbent insert being proximal to the front and/or back transversal edge to form transversal waist barrier(s) (5) or wherein the bonding means comprise mechanical bonding preferably ultrasonic bonding

According to an embodiment, the method further comprises the steps of arranging an elastic material (12) over said discrete layer or patch (18) prior to step of folding an outer nonwoven layer (10) of the front and/or back panels (2, 3) over the folded absorbent insert (4) and patch or (18).

According to an embodiment, the method further comprises the steps of folding an outer nonwoven layer (10) of the front and/or back panels (2, 3) over the absorbent insert (4) and discrete layer and joining said folded outer nonwoven layer (10) directly or indirectly to the absorbent insert (4) partially covered by the discrete layer.

Further methods herein for the manufacture of an absorbent article 1 may comprise the steps of: providing a front panel 2; providing a back panel 3; providing an absorbent insert 4 comprising front and back transversal edges and left and right longitudinal edges connecting the front and back transversal edges to form a perimeter thereof (and generally wherein the left and right longitudinal edges extend substantially along the longitudinal axis y and are oppositely disposed such that said longitudinal axis y extends therebetween, and the front and back transversal edges extend substantially along the transversal axis x, and typically substantially perpendicular to the longitudinal axis y, and are oppositely disposed such that said transverse axis x extends therebetween); folding a portion of said absorbent insert 4 proximal to the front and/or back transversal edges thereof onto itself, preferably the fold being in the shape of a substantially U-fold, to form transversal waist barrier(s) 5; joining said insert 4 to said front and back panels 2, 3 (generally such that at least a portion of the absorbent insert 4 overlaps each of the front and back panels 2, 3 and forms a connection bridge between said front and back panels); and joining said front and back panels 2, 3 together along a pair of oppositely disposed side seams. Advantageously an absorbent article having transversal barrier(s) as described herein can be attained in a simple yet effective manner.

Generally the front and back panels herein are made from continuous webs that are subsequently severed into discrete front and back panels typically after the step of joining the absorbent insert 4 to said front and back panels 2, 3.

The above method is not limited to this sequence of steps and the present disclosure encompasses different sequence of steps. For example, a method for the manufacture of an absorbent article 1 may comprise the steps of: providing an absorbent insert 4 comprising front and back transversal edges and left and right longitudinal edges connecting the front and back transversal edges to form a perimeter thereof; folding a portion of said absorbent insert 4 proximal to the front and/or back transversal edges thereof onto itself, preferably in the shape of a substantially U-fold, to form transversal waist barrier(s) 5; providing a front panel 2; providing a back panel 3; joining said insert 4 to said front and back panels 2, 3; and joining said front and back panels 2, 3 together along a pair of oppositely disposed side seams.

Preferably, said folding step is carried out by static folding where a static element, *i.e.* a stator, such as a stationary folding blade deflects a portion of said absorbent insert 4 proximal to the front and/or back transversal edges thereof onto itself. In other words, the folding is step is preferably done by passive folding meaning that the folding is done with a moving element, i.e. the absorbent insert, being folded by an unmoving, or immobile or fixed element, e.g. a blade. Such folding is more cost efficient and more reproducible. The present disclosure encompasses embodiments where the folding step is carried out by dynamic folding, where a mobile element, e.g. an actuated folding blade, moves or is actuated to fold a portion of said absorbent insert 4 proximal to the front and/or back transversal edges thereof onto itself.

Preferably, the method further comprises the steps of folding an outer nonwoven layer 10 of the front and/or back panels 2, 3 over the folded absorbent insert 4 and/or an inner nonwoven layer 9 and joining said folded outer nonwoven layer 10 directly or indirectly to the folded absorbent insert 4. Advantageously this allows to prevent irritation or discomfort to the wearer as explained hereinabove whilst avoiding the use of added components/materials/elements whilst maintaining a simple and cost-effective production process.

Preferably, the method further comprises the step of arranging an elastic material 12 over the folded portion of the folded absorbent insert 4 prior to step of folding an outer nonwoven layer 10 of the front and/or back panels 2, 3 over the folded absorbent insert 4.

Preferably, the method further comprises the step of intermittently applying adhesive onto said portion of the absorbent insert proximal to the front and/or back transversal edges.

The adhesive may be applied in joining zones or bonding zones. The joining or bonding zones comprises at least one area where the adhesive or mechanical bonding mean is applied.

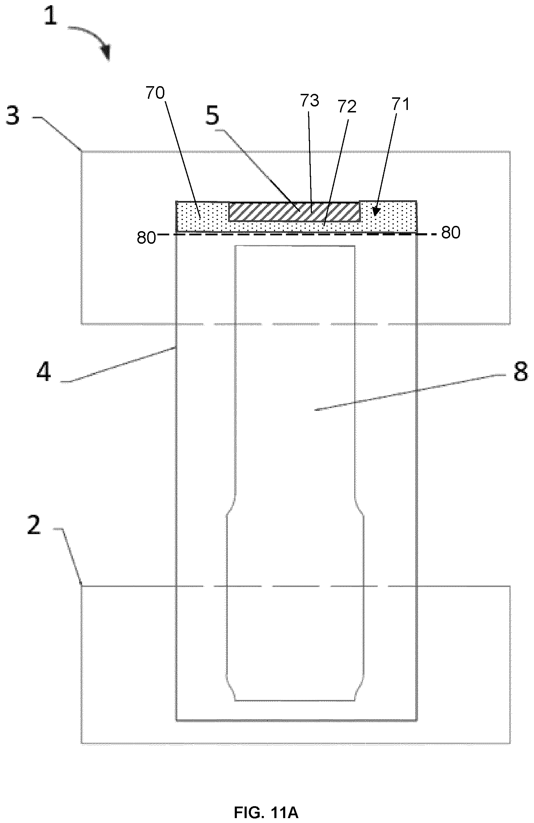

According to an embodiment as illustrated in FIG. 11A, which represents an absorbent article 1 prior to the folding step of the absorbent insert 4, the adhesive is applied in a pattern 71 on one portion of said absorbent insert 4 proximal to the back transversal edge. FIG.11B illustrates the same absorbent article 1 after the folding step where the portion of the absorbent insert 4 proximal to the back transversal edge is folded thereof onto itself. The adhesive can also be applied in such pattern 71 to one portion of said absorbent insert 4 proximal to the front transversal edge. Naturally, the adhesive can be applied in such pattern 71 to both portions of said absorbent insert 4 proximal to the front and back transversal edges. The pattern 71 of adhesive comprises the two portions or two areas of adhesive 70, meaning two joining zones, as described hereabove, meaning the area 70 at the transversal extremities of the transversal edge, or in other words, the corners of the absorbent insert 4, or also the portions where the back transversal edge and longitudinal edges of the absorbent insert 4 connect as illustrated in FIG. 11A. When applied, the pattern 71 of adhesive further comprises a further area 72 of adhesive extending transversally connecting the two areas 70 and an area free of adhesive 73, said area free of adhesive 73 is arranged between the transversal edge of the absorbent insert 4 and the further area 72 of adhesive with respect to the longitudinal direction (y) and arranged between the two areas 70 at the corners with respect to the transversal direction (x). In other words, the pattern 71 of adhesive comprise a U-shaped or C-shaped pattern with two areas 70 corresponding to the upper branches of the U, said areas 70 being connected by a further area 72 corresponding to the lower branch of the U. Similarly, the pattern 71 of adhesive comprise a H-shaped pattern with two areas 70 corresponding to the side branches of the H, said areas 70 being connected by a further area 72 corresponding to the middle branch of the H. The pattern of adhesive can comprise two areas 70 at the corners and a further area 72 of adhesive which extend only partially along the transversal axis (x) and does not connect the two areas 70 while still defining an area free of adhesive 73 as described above. In other words, the further area 72 can comprise a gap free of adhesive. In other words, the pattern 71 of adhesive can comprise two areas 70 that are L shaped in facing relationship or in mirror relationship, said L-shaped areas being arranged at each corner.

Generally, with an adhesive pattern 71 as described hereabove, when folding the absorbent insert 4 along the folding line 80 illustrated in FIG. 11A or FIG.11B, a transversal waist barrier 5 is defined with the area free of adhesive 73 not being joined to the absorbent insert 4 and therefore standing upright. In other words, said barrier(s) 5 herein form an opening, typically about a terminal edge of the folded outer nonwoven layer, of an exudate containing pocket wherein substantially the entire surface of the pocket being congruent with a topsheet 6 of the absorbent insert 4 comprises a liquid impermeable layer preferably being a film, more preferably an elastic film (generally according to the embodiments described herein). With such adhesive pattern 71, the angle defined by the fold and the waist barrier is more acute and can provide a transversal waist barrier 5 with more resistance to exudates.

According to an embodiment, the method can comprise a mechanical bonding step where the portion of the absorbent insert 4 proximal to the back transversal edge is folded thereof onto itself and then mechanically bonded. The method comprises a step where the portion of the absorbent insert 4 proximal to the back transversal edge is glued to the absorbent insert 4 and/or a step where the portion of the absorbent insert 4 proximal to the back transversal edge is mechanically bonded to the absorbent insert 4. For example, adhesive is intermittently applied to the portion of the absorbent insert 4 proximal to the back transversal edge, then said portion is folded thereof onto itself, then the folded portion is mechanically bonded, e.g. ultrasonic welding, to further secure the transversal waist barrier 5.

According to another embodiment, the method for the manufacture of an absorbent article (1) comprises the steps of:

- providing a front panel (2);
- providing a back panel (3);
- providing an absorbent insert (4) comprising front and back transversal edges and left and right longitudinal edges connecting the front and back transversal edges to form a perimeter thereof;
- applying a pattern of adhesive onto a discrete layer or patch preferably comprising liquid impermeable film, e.g. a patch of liquid impermeable film, and/or applying a pattern of adhesive onto a portion of the absorbent insert (4) proximal to the front and/or back transversal edge said pattern of adhesive extending all along the transversal edges and partially along the longitudinal edges of the patch and/or absorbent insert (4) and applying said discrete layer or patch preferably comprising liquid impermeable film onto the top surface of the absorbent insert (4) at a portion of the absorbent insert being proximal to the front and/or back transversal edge to form transversal waist barrier(s) (5);

and/or

- applying a discrete layer or patch preferably comprising liquid impermeable film, e.g. a patch of liquid impermeable film onto
- joining said insert (4) to said front and back panels (2, 3); and
- joining said front and back panels (2, 3) together along a pair of oppositely disposed side seams.

The above method is not limited to this sequence of steps and the present disclosure encompasses different sequence of steps. For example, the method for the manufacture of an absorbent article (1) comprising the steps of: providing a front panel (2); providing a back panel (3); providing an absorbent insert (4) comprising front and back transversal edges and left and right longitudinal edges connecting the front and back transversal edges to form a perimeter thereof; joining said insert (4) to said front and back panels (2, 3); applying a pattern of adhesive onto a discrete layer or patch preferably comprising liquid impermeable film, e.g. a patch of liquid impermeable film, and/or applying a pattern of adhesive onto a portion of the absorbent insert (4) proximal to the front and/or back transversal edge said pattern of adhesive extending all along the transversal edges and partially along the longitudinal edges of the patch and/or absorbent insert (4); applying said discrete layer or patch (18) preferably comprising liquid impermeable film onto the top surface of the absorbent insert (4) at a portion of the absorbent insert being proximal to the front and/or back transversal edge to form transversal waist barrier(s) (5) and joining said front and back panels (2, 3) together along a pair of oppositely disposed side seams.

Preferably, this embodiment further comprises the step of arranging an elastic material (12) over said discrete layer or patch preferably comprising liquid impermeable film prior to step of folding an outer nonwoven layer (10) of the front and/or back panels (2, 3) over the folded absorbent insert (4) and patch.

Said discrete layer or patch (18) preferably comprising liquid impermeable film is preferably in the form of a rectangular patch. Said rectangular patch extends along the transversal edge of the absorbent insert (4) and partially along the longitudinal edges of the absorbent insert (4) as illustrated in FIG. 10. In other words, said patch (18) covers the portion of the absorbent insert (4) proximal to the front and/or back transversal edge, preferably the portion of the absorbent insert (4) proximal to the back transversal edge.

In an embodiment, the discrete layer or patch (18) preferably comprising liquid impermeable film is bonded, meaning joined or associated, to the absorbent insert (4) by at least one bonding means (19). Bonding means comprise and are not limited to for example adhesive bonding and/or mechanical bonding and/or ultrasonic bonding. The discrete layer or patch (18) can be joined to the absorbent insert (4) two or more bonding means. As illustrated in FIG. 10, the bonding means comprises here adhesive bonding (19a) in an area arranged along the transversal edge of the absorbent insert (4) and mechanical bonding (19b) in an area arranged along the longitudinal edge of the portion of the absorbent insert (4) proximal to the front and/or back transversal edge, preferably the back transversal edge. According to another embodiment, the bonding means comprise only adhesive bonding (19a) arranged along the transversal and longitudinal edges of the portion of the absorbent insert (4) proximal to the front and/or back transversal edge. Arranging the bonding means at the transversal and longitudinal edges of the absorbent insert (4) enables to have a proper waist transversal barrier (5) and contain any exudate within the absorbent insert (4).

According to an embodiment, the total surface area of the discrete layer or patch (18) preferably comprising liquid impermeable film is greater than the surface area of the bonding means (19), meaning that at least a portion of the total surface area of the discrete layer or patch (18) preferably comprising liquid impermeable film is not joined to the absorbent insert. In other words, the bonding means extend over a surface area of the discrete layer or patch (18) preferably comprising liquid impermeable film that is lesser than the total surface area of the discrete layer or patch (18) preferably comprising liquid impermeable film.

According to an embodiment, the adhesive (19a) can be applied in a pattern, such as in a U-shaped or C-shaped pattern extending along the transversal and longitudinal edges of the portion of the absorbent insert (4) proximal to the front and/or back transversal edge. Such pattern can optionally comprise notches 19c to save on raw material.

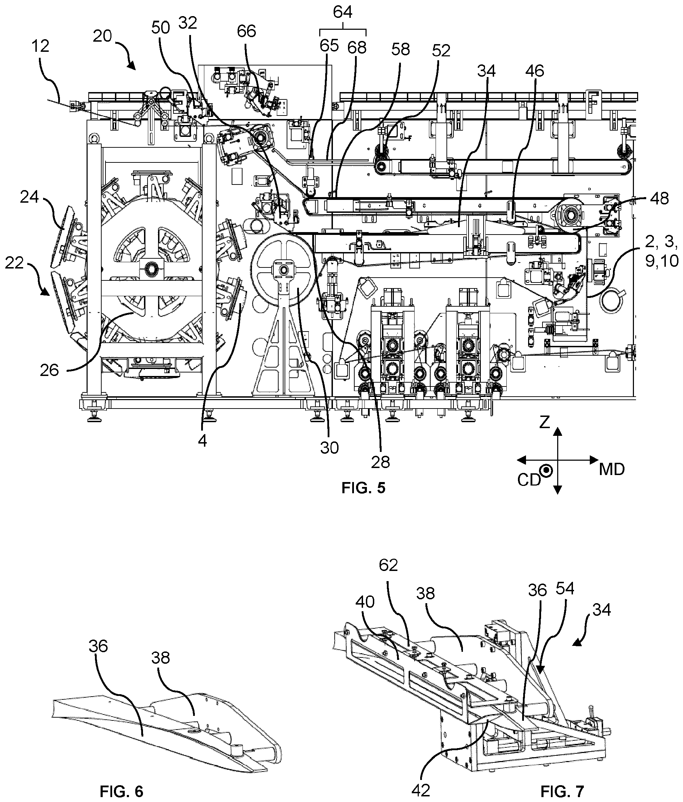

An apparatus 20 may be used to manufacture an absorbent article 1 as described herein and following a method disclosed herein. Figure 5 illustrates one possible embodiment for an apparatus 20 for manufacturing such absorbent articles 1. The apparatus 20 extends in a machine direction MD, a cross-direction CD and a vertical direction Z. The machine direction MD corresponds to the direction of travel of the webs, elastic materials or any components of the absorbent articles 1 being manufactured, the cross-direction CD is a direction perpendicular to the machine direction MD, and the vertical direction Z correspond to the height and is also a direction perpendicular to both the machine direction MD and the cross-direction CD. In other words, the machine direction MD is the flow direction of an absorbent article and its components being assembled in the process line, e.g. the machine direction MD is the direction in which the absorbent insert 4 is conveyed. The terms "upstream" and "downstream" as used herein are with respect to the machine direction MD.

For sake of clarity, the coordinate system using longitudinal and transversal axis (x,y) pertains to the absorbent article 1 alone e.g. when laid flat on a surface, whereas coordinate system using machine, cross and vertical directions

**EP 4 458 337 B1**

(MD, CD, Z) pertains to the apparatus for manufacturing absorbent article 1 with the absorbent articles 1 and their components being conveyed.

Following the method described herein above, an absorbent insert 4 may be provided. Here, the absorbent insert 4, assembled beforehand, may be transferred by a transfer device 22, a unit 22 comprising a plurality of transfer heads 24 arranged on a support wheel 26 with both the support wheel 26 and the transfer heads 24 being configured to respectively rotate around a respective axis. The transfer device 22 is generally an apparatus that is configured to turn and repitch the discrete absorbent inserts 4, turn the discrete absorbent inserts 4, or merely transfer the discrete absorbent inserts 4 between a pick-up location and a drop-off location. The transfer device 22, as illustrated in FIG. 5, comprises a frame 26, here a support wheel 26, defining a rotation axis and a plurality of transfer heads 24 associated to said frame 26. The transfer heads 24 are configured to circumnavigate about the rotation axis in an orbit. As illustrated in FIG. 5, the support wheel 26 rotates around an axis parallel to the cross-direction CD and the transfer heads 24 circumnavigate about this rotation axis in an orbit. The orbit passes through the pick-up location and the drop-off location. The transfer device 22 turns the discrete absorbent inserts 4 in any suitable angle. For example, as illustrated in FIG. 5, the transfer device 22 turns the discrete absorbent inserts 4 about 90 degrees. The discrete absorbent inserts 4 are turned by the transfer heads 24 turning. For instance, a transfer head 24 may pick up a discrete absorbent insert 4 in a pick-up location, turn 90 degrees about an axis perpendicular to the rotation axis of the frame, e.g. about the vertical direction Z, drop off the discrete absorbent insert 4 in a drop-off location, and the turn back to its original position (either in the same direction or an opposite direction) before orbiting back through the pick-up location. In other words, the transfer device 22 transfers an absorbent insert 4 extending in the machine direction (MD) and rotates it so that said absorbent insert 4 extends in the cross direction (CD).

The absorbent insert 4 may be deposited onto a first conveying device 28, e.g. a conveyor belt 28, and/or a rotating drum 30. As illustrated in FIG. 5, the absorbent insert 4 is deposited onto a rotating drum 30, but said insert 4 can be directly deposited onto a first conveyor belt 28. The first conveyor belt 28 and/or rotating drum 30 is/are associated with a negative pressure device such that the absorbent insert 4 when deposited onto the belt 28 and/or drum 30 is sucked onto the first conveyor belt 28 and/or rotating drum 30 and is maintained onto said conveyor belt 28 and/or rotating drum 30 by vacuum. In other words, the first conveyor belt 28 and/or rotating drum 30 comprises vacuum holes on its outer surface, meaning a foraminous outer surface, and the absorbent insert 4 is deposited onto said outer surface. The absorbent insert 4 is maintained on the foraminous drum 30 and/or foraminous first conveyor belt 28 by the negative air-pressure generated by the suction. As illustrated in FIG. 5, the absorbent insert 4 is first deposited onto the rotating drum 30 and then transferred to the first conveyor belt 28, the absorbent insert 4 is then conveyed by the first conveyor belt 28 in the machine direction MD.

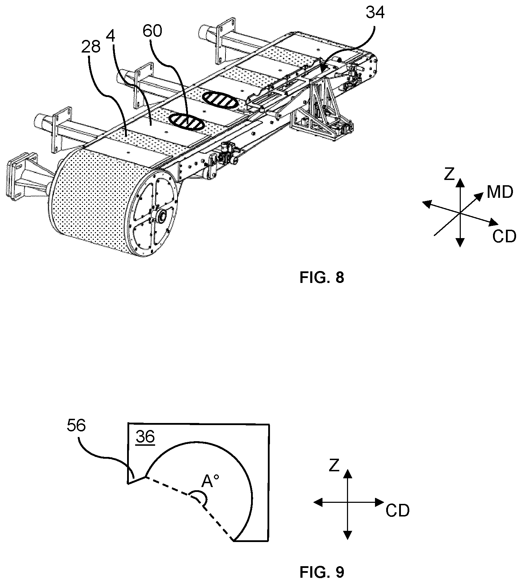

According to an embodiment, once being conveyed by the first conveyor belt 28, adhesive is applied intermittently on at least one transversal edges, namely the adhesive is applied intermittently on a portion of said absorbent insert 4 proximal to the front and/or back transversal edges, meaning on the portion described hereabove to be folded. In other words, the adhesive is applied intermittently on at least one edge arranged at one end of the absorbent insert 4 with respect to the cross-direction CD as illustrated in figure 8. The adhesive can be applied intermittently on one portion of said absorbent insert 4 proximal to the back transversal edges. The adhesive can be applied intermittently to one portion of said absorbent insert 4 proximal to the front transversal edges. The adhesive can be applied intermittently to both portions of said absorbent insert 4 proximal to the front and back transversal edges. The adhesive is applied intermittently such that the central area of said portion(s) proximal to the transversal edge(s) is not glued. In other words, the adhesive is applied on both portions 70 arranged at the corners of a transversal edge, but not in between said corners 70 as illustrated in FIG.4. For example, when considering FIG. 4, the adhesive is applied in the portion 70 located at the corner of the back transversal edge but is not applied on the portion where the absorbent core 8 extends, meaning when the back transversal edge and the absorbent core are transposed on a same machine direction axis, the adhesive applied is present at the corners but not where the absorbent core 8 extends. According to one embodiment, the adhesive is applied intermittently on one transversal edge only, preferably the back transversal edge. According to another embodiment, the adhesive is applied intermittently on both transversal edges. Alternatively, the adhesive can also be applied continuously in an adhesive pattern 71 as described above, for example in a U-shaped or a C-shaped or a H-shaped pattern.

The adhesive may be applied by a glue dispensing device 32. Such application of adhesive may be by various processes such as slot coating, spray coating and other topical applications using a pneumatic, piezo or electromagnetic actuator. For example, the glue dispensing device 32 comprises at least one nozzle, each nozzle, for instance, may comprise a slot coating device or several exit ports and an adhesive flow control device such as electromagnetic valve, e.g. a solenoid valve.

The absorbent insert 4 may then be conveyed, or transported, by the first conveyor belt 28 to a folding unit 34. The folding unit 34 comprises a folding blade 36 that is curved (FIG. 6,9) and that can be associated with a support plate 38 as illustrated in FIG. 6. The folding blade 36, or curved blade 36, comprises a blade with a curvature said curvature spanning over an angle from 0° to at least 200°. In other words, the curved blade comprises a concave portion, said concave portion varying along the machine direction and extending over an angle A° of at least 180°, preferably at least 250°, more

25

preferably at least 300°, meaning angle A° is preferably a reflex angle as illustrated in FIG. 9. For sake of clarity, the folding blade 36 comprises a curve blade 36, said curved blade 36 defines an angle A° that evolves along the machine direction MD, meaning that for a given point in the machine direction MD, the folding blade 36 extends in the vertical and cross directions Z,CD as illustrated in FIG. 9, the two extremities of said blade 36 defining an angle A°, or folding angle A°, since the folding wall, meaning the wall used for folding the absorbent insert 4, of the blade is curved. In the upstream portion of the folding blade, the angle A° defined by the two extremities of the blade 36 is small, e.g. between 0° and 10°. The angle A° increases along the machine direction MD and is greater than 180° at the downstream portion of the folding blade 36. The support plate 38 is fixed, or removably associated to the folding blade 36. Having a blade 36 with a folding angle A° of 180° is sufficient to fold the absorbent insert 4 onto itself but it can lead to having wrinkles or a gap in the fold. Having a blade 36 with a folding angle A° of 250° or more enables to have a smoother fold and reduces the risks of wrinkles or a gap in the fold.

Alternatively or in combination, the apparatus 20 can comprise a depositing unit, for example a liquid impermeable film layer depositing unit, or a patch depositing unit or a patch applying unit where a plurality of liquid retarding layers in the form of a patch or a discrete layer preferably of liquid impermeable film applied in the form of a patch is joined to said absorbent insert (4) via suitable bonding techniques such as adhesive and/or mechanical bonding as described hereabove. The apparatus comprises a bonding unit to join the patch 18 to the absorbent insert (4) and/or to the inner nonwoven layer (9), more generally to the back and/or front panel (3), to form a transversal barrier. The patch depositing unit and the bonding unit as sequential, in the sense that if the bonding means correspond to adhesive bonding, either the absorbent insert and/or the patch (18) pass first through the bonding unit first and adhesive is applied on the absorbent insert and/or the patch (18) and then through the patch depositing unit, alternatively, if the bonding means correspond to mechanical bonding the absorbent insert (4) passes through the patch depositing unit first and then through the bonding unit. The same applies for the embodiments where the patch is bonded to the front and/or back panel (3), specifically the inner nonwoven layer (9).

The folding unit 34 can further comprise a pressing blade 40 arranged at a distance d from the curved blade 36 with respect to the cross-direction CD and/or vertical direction Z. In other words, there is a gap between the curved blade 36 and the pressing blade 40 in the cross-direction CD and/or vertical direction Z. The pressing blade 40 presses vertically against the absorbent insert 4 and maintain said absorbent insert 4 in place, at least vertically. The combination of the curved blade 36 folding the portion of the absorbent insert 4 proximal to the front and/or back transversal edge, and the pressing blade 40 pushing the absorbent insert 4 at least vertically, ensures a proper folding of the portion of the absorbent insert 4 proximal to the front and/or back transversal edges thereof onto itself, in order words a proper folding of the portion of the absorbent insert 4 proximal to the front and/or back transversal edge to form a transversal barrier 5 as described herein. In other terms, the folding blade 36 deviates the portion of the absorbent insert 4 proximal to the front and/or back transversal edge in the cross direction CD and in the vertical direction Z and the pressing blade 40 deviates a portion of the absorbent insert 4, which is more distal to the transversal edge than said portion of the absorbent insert 4 proximal to the transversal edge e.g. in between said portion of the absorbent insert 4 proximal to the front and/or back transversal edge and the center of absorbent core 8, in the vertical direction Z. Simply put, the pressing blade 40 at least maintains the absorbent insert 4 in place while the folding blade 36 is folding the portion of the absorbent insert 4 proximal to the transversal edge. The folding blade 36 alone can suffice to fold the portion of the absorbent insert 4 proximal to the front and/or back transversal edge, and adding a pressing blade 40 further improves the folding. The folding blade 36 ensures a folding preferably in the shape of a substantially U-fold, to form transversal waist barrier(s) 5. In the embodiment described above where adhesive is applied intermittently at the corners 70, then only the corners 70 of the portion of the absorbent insert 4 proximal to the front and/or back transversal edges are folded and glued thereof onto itself.

According to an embodiment, in order to properly fold the portion of the absorbent insert 4 proximal to the front and/or back transversal edge thereof onto itself, the pressing blade 40 is preferably shorter in length, with respect to the machine direction MD, than the folding blade 36 so that when the curved blade 36 is folding the portion of the absorbent insert 4 proximal to the front and/or back transversal edge, namely once said portion has been folded by an angle of more than 180°, it folds thereof onto itself, meaning onto the upper surface of the absorbent insert 4, i.e. the topsheet 6.

According to an embodiment, the pressing blade 40 can be a flat plate as illustrated in FIG. 7, or it can be a rod or a cylindric tube. The pressing blade 40 can comprise an anti-adhesive coating to ensure that the absorbent insert 4 when being folded does not adhere onto the pressing blade 40. The pressing blade 40 can also be connected to an air-compressor and comprise air-vents or holes on its surface to blow out pressurized air to ensure that the absorbent insert 4 when being folded does not adhere onto the pressing blade 40.

According to an embodiment, the pressing blade 40 can comprise a sloped portion 42 at the upstream portion of the pressing blade 40 with respect to the machine direction MD as illustrated in FIG. 7. The slope portion 42 extends diagonally downward while extending downstream for holding down the absorbent insert 4 and improving the folding as described hereabove.

Once the at least one portion of the absorbent insert 4 proximal to front and/or back transversal edge has been folded thereof onto itself thereby forming a transversal waist barrier 5, the absorbent insert 4 can be conveyed toward one or more pressure roller(s) 46 which is arranged to press the folded portion, meaning the transversal waist barrier(s) 5, thereby securing the fold and eventually securing the glued and/or welded portions together.

The partially folded absorbent insert 4 may then be guided toward a rotating drum 48 where it is deposited onto two webs corresponding to front and back panels 2,3 comprising the inner nonwoven layer 9 and outer nonwoven layer 10 as described hereabove. The front and back panels 2,3 are assembled beforehand with the lamination of inner and outer nonwoven layers 9,10 and elastic material 11 and by intermittently severing the elastic material 11.

The partially folded absorbent insert 4 and front and back panels 2,3 may then be guided, for example by a second conveying device 58, such as a conveyor belt, to an elastic depositing unit or station, where a second elastic material 12, e.g. one or more elastic strands 12, is deposited onto the backsheet 7 or the outer cover 7' of the absorbent insert 4 at the region that has been folded over. The elastic strand(s) 12 is typically beforehand coated with adhesive by an adhesive dispensing unit 50 such that when the elastic strand(s) 12 is applied on the backsheet 7 or outer cover 7' on the portion of the absorbent insert 4 that has been folded thereof onto itself, it remains in place. The adhesive is preferably continuously applied on the elastic strand(s) 12. In other words, the elastic strands 12 in any of the embodiments herein may be strand-coated with adhesive prior to joining to one or more layers such as the inner and outer nonwoven layers 9, 10 to form elastic panels 2, 3 and/or the backsheet 7, outer cover 7' and/or outer nonwoven layer 10 to form the elastic transversal waist barrier(s) 5. Alternatively or in combination, the elastic strand(s) 12 can be applied on the patch or discrete layer (18).

Preferably the elastic material 12, e.g. the elastic strand(s) 12, is arranged such that said elastic material 12 is sandwiched between the absorbent insert 4 and the outer nonwoven layer 10. More preferably, the absorbent insert 4 as described herein comprises a backsheet 7 and eventually an outer cover 7' on the garment facing surface of the absorbent core 8. The present disclosure encompasses embodiments where the elastic material 12 is sandwiched between the backsheet 7 and the outer nonwoven layer 10, embodiments where the elastic material 12 is sandwiched between the outer cover 7' and the outer nonwoven layer 10 or even embodiments where the elastic material 12 is sandwiched between the backsheet 7 and outer cover 7' and the outer nonwoven layer 10 e.g. in cases where the outer cover 7' is transversally shorter than the backsheet 7. In other words, the elastic material 12 is directly or indirectly joined to the backsheet 7, preferably by adhesive, and sandwiched between the backsheet 7 and/or outer cover 7' and the outer nonwoven layer 10.

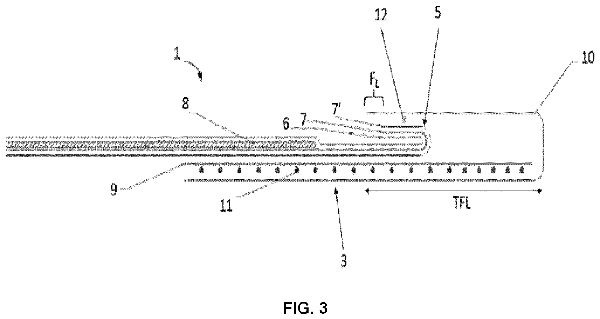

The outer nonwoven layer 10 may then be folded over the elastic strand 12, backsheet 7, outer cover 7' and/or the transversal elastic barrier 5 and by a second folding apparatus 64, in order to form the absorbent article 1 as illustrated in FIG. 3. The apparatus 20 can further comprise an additional adhesive dispensing device 66 to apply adhesive on the portion of the front and/or back panels 2,3 to be folded. The apparatus 20 can further comprise a pressure roller 52 to apply pressure on the transversal edges of the front and/or rear panels 2,3 and ensure the structural integrity of the chassis. Preferably the outer nonwoven layer 10 is folded over the absorbent insert and/or thereof onto itself by the second folding apparatus 64 at the front and back panels 2,3 and the additional adhesive dispensing device 66 applies adhesive on a portion of the absorbent insert 4 proximal to the transversal edges of the front and back panels 2,3. The second folding apparatus 64 comprises a roller 65 that is arranged to cover a portion of the front and/or back panels 2,3, namely the roller is arranged at a distance from the transversal edge of the front and/or back panel 2,3 so that said roller deviates vertically said portion of the front and/or back panels 2,3, the transversal edge of the front and/or back panel which is not deviated by the roller naturally folds upward. The folding apparatus 64 can further comprise a railing 68 oriented diagonally with respect to the cross-direction CD toward the center of absorbent insert 4 to guide and facilitate the folding of the non-deviated portion of the outer nonwoven layer 10, i.e. the transversal edge of the front and/or back panel 2,3 onto the absorbent insert 4, namely onto the transversal barrier 5, backsheet 7 and/or outer cover 7'.

The belt, meaning the front and back panels 2,3 may then be cut in the cross-direction CD into individual pieces, each individual piece comprising an absorbent insert 4 with a front and back panel 2,3. Each individual piece is then folded to bring the front and back panels 2,3 in proximity of one another and then associated in side seams to form an individual absorbent article 1 with a transversal barrier 5. The front and back panels 2,3 are joined, or associated, by bonding said panels 2,3 together at a bonding unit. The bonding unit can be selected from and not limited to an adhesive dispensing device, an ultrasonic device or a welding device. Alternatively, the front and back panel 2,3 can be joined first and then cut in the cross direction into individual pieces.

According to an embodiment, the folding unit 34 comprises a support device 54 to carry the folding blade 36 and/or support plate 38 and/or pressing blade 40. The support device 54 can comprise means to move the folding blade 36 and/or support plate 38 and/or pressing blade 40, e.g. a system with rails and an actuator to slide the folding blade 36 and/or support plate 38 and/or pressing blade 40 in the cross-direction CD and/or vertical direction Z. The support device 54 can comprise means to remove the folding blade 36 and/or support plate 38 and/or pressing blade 40, e.g. a plurality or screws, bolts, threaded rods and nuts, etc. so that the folding blade 36 and/or support plate 38 and/or pressing blade 40 can be removed entirely. Such system enables to easily change the production from an absorbent article 1 with transversal barrier(s) 5 to a standard product without transversal barrier(s) 5. The adhesive dispensing device and elastic strand(s) 12 guiding device are turned off for standard products. Alternatively, the adhesive dispensing device 32 can continuously apply adhesive.

According to an embodiment, the folding blade 36 comprises an extension 56 as illustrated in FIG. 9 where the extension 56 extends beyond the curvature of the blade 36 to ensure that the transversal edge is well folded thereof onto itself.

According to an embodiment, the first conveyor belt 28 comprises a foraminous portion arranged at the central portion 60 of the first conveyor belt 28 with respect to the cross-direction CD as illustrated in FIG. 8. Such arrangement ensure that the absorbent insert 4 is well fastened in the central portion 60, meaning in the crotch region of the absorbent insert 4, and is loosely secured at the at least one portion of the absorbent insert 4 proximal to the transversal edge. Such arrangement ensures that the folding blade 36 does not meet any resistance and that the portion of the absorbent insert 4 proximal to the transversal edge can be folded more easily. In other words, the vacuum should not impede the folding at the one or both cross direction extremities. The first conveyor belt 28 can also comprise a portion blowing out air at one cross direction extremity, the extremity proximal to the folding unit 34, to favor the lifting and thus folding of the portion of said absorbent insert 4 proximal to the front and/or back transversal edge.

According to an embodiment, the folding unit 34 comprises an adjustable mechanism 62 to adjust the distance between the pressing blade 40 and the folding blade 36. Such adjustable mechanism comprises for example a plate with notches and screws enabling the sliding of the plate between the notches and adapt the distance with respect to the cross direction and/or vertical direction.

According to an embodiment, the absorbent insert 4 associated with, or deposited onto, the front and back panels 2,3 are conveyed from the rotating drum 48 to the elastic strand(s) 12 application unit by a second conveying unit 58, e.g. a second conveyor belt 58. The first conveyor belt 28 and the second conveyor belt 58 can transport the absorbent inserts 4 at the same speed to avoid the formation of wrinkles or creases. The first conveyor belt 28 and the transfer device 22 can also rotate at matching speed to avoid the formation of wrinkles or creases.

According to an embodiment, the two webs of front and back panels can be conveyed at same speed or at different speed. If the two webs of front and back panels are conveyed at same speed, front and back panels will have the same lengths with respect to the transversal direction or machine direction and the side seams will be arranged at the sides of the wearer. If the two webs of front and back panels are conveyed at different speed, e.g. the web of back panel is conveyed faster than the web of front panel then the back panel will be longer than the front panel and the side seams will be more toward the front at the sides of the wearer.

According to an embodiment, the folding unit 34 comprises two folding blades 36 each with eventually with a pressing blade 40, one arranged at each transversal edge of the absorbent insert 4, meaning one arranged at each end of the first conveyor belt 28 with respect to the cross-direction CD. The two folding blades 36 are substantially identical in dimensions and shape and are preferably arranged in facing relationship or in mirror relationship, meaning at the same area with respect to the machine direction MD. Such arrangement enables to balance the force exerted onto the absorbent insert 4 and optionally to remove the pressing blade(s) 40. The rest of the process is arranged accordingly, namely an elastic thread(s) 12 is arranged between the absorbent insert 4 and the front panel which is folded onto the elastic thread(s) 12. Such arrangement also enables to have a transversal waist barrier 5 in the front and in the back.

According to an embodiment the elastic material 11 sandwiched between the inner nonwoven layer 9 and the outer nonwoven layer 10 and the elastics strands 12 are different. The elastics 11,12 can differ in cross-sectional shapes, e.g. round or flat, in dtex, in composition and/or in color. Alternatively, said elastic material 11 and elastic strands 12 can the same material.

The present disclosure is not limited to these embodiments, the method and process can comprise the placing of leg cuffs and leg elastics, wetness indicators, etc. The different embodiments can also be combined. For example it is possible to arrange a patch 18 onto the absorbent insert (4) and further fold a portion of said absorbent insert (4) proximal to the front and/or back transversal edges thereof onto itself. In other words, the embodiments where the transversal waist barrier(s) (5) is a patch or a folded portion of the absorbent insert (4) are not mutually excluding one another and they can be combined and features described in relation to one can be applied to the other.

<u>Wrinkle distribution determination</u>

The wrinkle distribution herein is determined by the following method.

For each side panel to be tested, the number of wrinkles is counted over a length of 10mm along the panel length (L) with the laminate in relaxed state (i.e. without applying an extension force between ends thereof, preferably taken at its "unused" state wherein "unused" herein means without the panel ever having been previously stretched since manufacture). The total number of wrinkles is then divided by 10 in order to provide the number of wrinkles per unit length. A total of 4 random locations within the elastic region (20) of the laminate may be measured accordingly and an average is calculated for each sample.

The 10mm length is typically taken from a starting position that encompasses at least one full wrinkle (i.e. a complete wrinkle comprising a peak positioned between two consecutive troughs) and by doing so if at the opposite extremity of the 10mm length only a partial wrinkle (i.e. not a complete wrinkle comprising a peak positioned between two consecutive troughs, e.g. the 10mm end at a position corresponding to the peak of a wrinkle) is formed, this is not counted as a wrinkle within the present method.

The above procedure is typically repeated for at least 4 samples of side panels and an average is calculated to provide the wrinkle distribution in wrinkles/mm as referred to herein.

Hysteresis Test Method

The Hysteresis Test can be used to various specified strain values. The Hysteresis Test utilizes a commercial tensile tester (e.g., from Instron Engineering Corp. (Canton, MA), SINTECH-MTS Systems Corporation (Eden Prairie, MN) or equivalent) interfaced with a computer. The computer is used to control the test speed and other test parameters and for collecting, calculating, and reporting the data. The tests are performed under laboratory conditions of 23 °C + 2°C and relative humidity of 50% + 2%. The specimens are conditioned for 24 hours prior to testing.

The specimen is cut with a dimension of 10 mm in the intended stretch direction of the ear X 25.4 mm in the direction perpendicular to the intended stretch direction of the ear. A specimen is collected from either an inelastic region or from an elastic region.

1. Select the appropriate grips and load cell. The grips should have flat surfaces and should be wide enough to grasp the specimen along its full width. Also, the grips should provide adequate force and suitable surface to ensure that the specimen does not slip during testing. The load cell is selected so that the tensile response from the specimen tested is between 25% and 75% of the capacity of the load cell used.

2. Calibrate the tester according to the manufacturer's instructions.

3. Set the distance between the grips (gauge length) at 7 mm.

4. Place the specimen in the flat surfaces of the grips such that the uniform width lies along a direction perpendicular to the gauge length direction. Secure the specimen in the upper grip, let the specimen hang slack, then close the lower grip. Set the slack preload at 5 gram/force. This means that the data collection starts when the slack is removed (at a constant crosshead speed of 13mm/min) with a force of 5 gram force. Strain is calculated based on the adjusted gauge length (lini), which is the length of the specimen in between the grips of the tensile tester at a force of 5 gram force. This adjusted gauge length is taken as the initial specimen length, and it corresponds to a strain of 0%. Percent strain at any point in the test is defined as the change in length relative to the adjusted gauge length, divided by the adjusted gauge length, multiplied by 100.

5(a) First cycle loading: Pull the specimen to the 100% strain at a constant cross head speed of 70 mm/min. Report the stretched specimen length between the grips as Imax.

5(b) First cycle unloading: Hold the specimen at the 100% strain for 30 seconds and then return the crosshead to its starting position (0% strain or initial sample length, lini) at a constant cross head speed of 70 mm/min. Hold the specimen in the unstrained state for 1 minute.

5(c) Second cycle loading: Pull the specimen to the 100% strain at a constant cross head speed of 70 mm/min.5(d) Second cycle unload: Next, hold the specimen at the 100% strain for 30 seconds and then return the crosshead to its starting position (i.e. 0% strain) at a constant cross head speed of 70 mm/min.

A computer data system records the force exerted on the sample during the test as a function of applied strain. From the resulting data generated, the following quantities are reported:

i. Length of specimen between the grips at a slack preload of 5 gram-force (Imi) to the nearest 0.001 mm.
ii. Length of specimen between the grips on first cycle at the 100% strain (Imax) to the nearest 0.001 mm.
iii. Length of specimen between the grips at a second cycle load force of 7 gram-force (lext) to the nearest 0.001 mm.
iv. % Set, which is defined as (lext - Imi) / (Imax - Imi) * 100% to the nearest 0.01%.

The testing is repeated for six separate samples and the average and standard deviation reported.

Breathability and WVTR test (or Moisture Vapor Permeability) method:

The moisture vapour (or vapor) permeability of a layer, or a composite laminated structure comprising a plurality of such layers, is measured via the Water Vapour Transmission Rate (WVTR) at 30°C and 15% relative humidity according to the ISO 15106-1:2003 test method. A Lyssy L80-5000 Water Vapor Permeation Analyser (manufactured and sold by Systech Instruments Ltd and Illinois Instruments Inc.) may be used.

The following modifications to the standard method are made: The test samples are clamped to a tensile testing machine (such as an Instron 5543 or similar) and stretched at a cross-head speed of 300mm/min at the desired elongation (such as 0%; 20%; 40%; 60%; 80%; 100%; 120%; and 140% elongations). As an example, a test sample having a length of 40mm at 0% elongation is stretched to 48mm length at 20% elongation; 56mm length at 40% elongation; 64mm length at 60% elongation; 72mm length at 80% elongation; 80mm length at 100% elongation; 88mm length at 120% elongation;

96mm length at 140% elongation; and so on.

For each elongation, the stretched test sample is then affixed to a self-adhesive sample card with an open area of 2.5cm2 which is to be covered by the specimen that is subsequently tested according to the WVTR method described in the above referenced standard.

The sample card with stretched film sample is inserted into the test chamber of the Water Vapor Permeation Analyser. The lower test chamber has a saturated atmosphere maintained by a small water reservoir, while the upper chamber contains a sensitive, fast-responding relative humidity sensor. The upper chamber is first dried to a defined humidity level using dry air.

When the drying is complete, the airflow stops and the valves close. From that point, the chamber is a closed system, in which transmission of water vapour through the samples causes an increase in relative humidity in the upper chamber, which is recorded by humidity sensor in the chamber. The Water Vapor Permeation Analyser measures the time required for the upper chamber humidity to increase from a pre-defined lower limit to a pre-defined upper limit. The measured time interval is compared to the time obtained during calibration with a standard film of known permeability (Goretex®) and the results is expressed as the water vapour transmission rate in g/m2/24h.

This test is repeated for different samples at each of the % elongations and results recorded. At least two WVTR (g/m2/24h) measurements are taken for each % elongation and an average WVTR is determined at each such elongation.

Seepage Index Test Method:

The Seepage is defined as a leakage fraction of artificial liquid exudates (saline solution composition described in the following sections for use in the test procedure herein) that is expelled beyond and/or through the transversal waist barrier or pocket of the absorbent article.

An absorbent article in the pant form (i.e. the type of absorbent articles to which the present invention generally refers) is cut open along the side seams.

Thereafter, the width of the back elastic belt is measured by applying a weight of 1.000 kg across the side edges formed along the previously cut open side seams of the back elastic belt. One of the side edges of the back elastic belt is clamped centered on the last centimeter of the side edge of the back elastic belt in a flat jaw 5 cm wide fixture with 1cm clamping depth. It is hung vertically and a 1.000kg weight is attached to an identical clamp that is provided the same manner and position on the other side edge of the back elastic belt and the weight is slowly released manually. After 30 seconds, the back elastic belt width is measured from clamp to clamp and 2cm are added to the result. The result is rounded to the next cm.

If the front elastic belt width is significantly different from the width of the back elastic belt (as can easily be assessed by simply stretching out the front and back elastic belt by hand), the width of the front elastic belt is determined in the same manner as the width of the back elastic belt.

The same procedure as described above is used for measuring the length of the absorbent article by applying a weight of 0.300kg. The absorbent article is clamped across the longitudinal centerline of the absorbent article on the last centimeter of the back waist edge in a flat jaw 15 cm wide fixture (centered on the longitudinal centerline) with 1cm clamping depth. It is hung vertically and a 0.300kg weight is attached to an identical clamp that is provided the same manner and position on the front waist edge of the absorbent article and the weight is slowly released manually. The absorbent article length is then measured after 30 second from clamp to clamp and 2cm are added to the result. The result is rounded to the next cm.

An inclined surface is made from acrylic plates so that a laying face corresponding to the face onto which an article is to be placed, is at an angle of 25° from a horizontal plane (e.g. a table onto which the inclined surface is placed). The laying face should be sized so as to accommodate thereon an absorbent article in laid-flat sate (i.e. stretched/extended to the above explained measured width and length dimensions). The whole laying face of the plate may be conveniently coated with hook material to nicely hold the absorbent article in laid-flat sate by interaction of said hooks with nonwoven layers of the article or alternatively tapes may be used to hold the article in position.

All testing is performed in a room controlled at 23°C ± 3°C and 50% ± 2% relative humidity. Samples are conditioned at 23°C ± 3°C and 50% ± 2% relative humidity at least two hours prior to testing.

The absorbent article is laid down in a flat planar configuration onto the inclined surface, on the laying surface of the inclined surface, with the topsheet facing upwards and the back sheet taut, extended to the before measured width and length dimensions and fixed with tape stripes or joined to the hook surface of the plate as explained above. The article being positioned such that the waist barrier is located closest to the horizontal plane with the opening thereof facing away from said horizontal plane (i.e. such that it is located towards the bottom of the inclined slope and the opening facing upwards so that liquid can be poured/added therein with the aid of gravity).

With the aid of a ruler and black marker, make marking points 5mm above the terminal transversal edge (TT) of the pocket. To avoid damaging or affecting the NW, mark the points outside the pocket and on the belt area at both sides if possible. A line 5mm above the terminal transversal edge (TT) of the pocket and running parallel therewith should correspond to an upper edge of a filter paper when placed according to the below explained procedure.

About 4g of saline solution (composition description below) is then added into the pocket/barrier with a pipette (preferably by applying it from left to right with a constant movement so that the liquid is evenly distributed in the pocket) and allow it to settle by waiting for 10 seconds.

Place one or more filter papers of a known weight such that the edge of the paper is aligned with the marking points as described above; and then position a weight of 4.2kg (+/-100g) thereon with an edge thereof aligned with the upper edge of the filter paper to hold the paper in place for a duration of 30 seconds.

Remove the weight (but leave the filter paper in place), wait for 10 seconds, and place the weight of 4.2kg (+/-100g) thereon again after which another about 4g of saline solution is added similar to the above explained procedure and followed by another waiting period of 30 seconds.

Repeat this procedure another two times so that the total amount of saline solution added is about 16g.

Liquid that leaks through the barrier is collected by the filter paper.

The filter paper is then weighed and the original (dry) weight is subtracted. This calculation a rewet value for the filter paper in grams (g).

$$\text{The Seepage Index (\%)} = (\text{rewet value (g)}/\text{total saline solution added (g)})*100$$

3 to 5 replicates on identical absorbent articles are tested. The reported Seepage Index (%) is reported as the average of the replicates and is calculated to within +/- 0.1%.

Saline solution preparation: 9g of NaCl are dissolved in 1000ml of $H_2O$.

**Claims**

1.  An absorbent article (1) for personal hygiene, preferably a disposable pant, comprising:

    - a substantially transversely extending front panel (2);
    - a substantially transversely extending back panel (3); and
    - a substantially longitudinally extending absorbent insert (4) joined to each of said front and back panels (2, 3), said absorbent insert (4) comprising front and back transversal edges and left and right longitudinal edges connecting the front and back transversal edges to form a perimeter thereof; and wherein said insert (4) comprises an absorbent core (8);

    wherein the front and back panels (2, 3) are joined together to define a pair of side seams, and **characterised in that** said article comprises a transversal waist barrier (5) proximal to the front and/or back transversal edges of the absorbent insert (4) wherein said barrier (5) comprises a plurality of liquid retarding layers wherein said plurality of liquid retarding layers comprise at least two layers selected from the group consisting of hydrophobic nonwovens, and substantially liquid impermeable films, and **in that** at least a majority of the waist barrier (5) comprises at least two of said plurality of liquid retarding layers and wherein said at least two of said plurality of liquid retarding layers are un-joined at one or more locations so that said layers are separated from one another at said location(s) by a gap (G), and preferably wherein said one or more locations together correspond to a majority of a total surface of said waist barrier (5).

2.  An absorbent article according to Claim 1 wherein the waist barrier (5) comprises at least two hydrophobic nonwovens intermittently joined together by adhesive, preferably by one or more elastic strands that are strand-coated with adhesive and sandwiched therebetween.

3.  An absorbent article according to any of the preceding Claims wherein the article has a Seepage Index of less than 35%, preferably is less than 30%, preferably less than 25%, more preferably less than 20%, even more preferably less than 10%, even more preferably from 0% to 5%, according to the Seepage Index Method herein.

4.  An absorbent article according to any of the preceding Claims wherein the hydrophobic nonwovens comprise, preferably consist of, synthetic fibers; preferably wherein said fibers comprise a material selected from polypropylene or derivatives thereof, polyethylene or derivatives thereof, polylactic acid or derivatives thereof, polylactic-co-glycolic acid or derivatives thereof, polyhydroxyalkanoates or derivatives thereof, and mixtures thereof.

5. An absorbent article according to any of the preceding Claims wherein the front and/or back panels (2, 3) comprise a plurality of elastic strands (11) sandwiched between two or more hydrophobic nonwoven layers, said nonwoven layers comprising at least an inner nonwoven (NW1) and an outer nonwoven (NW2) that are substantially congruent with each other to form a front and/or back panel laminate (PL); and wherein the front and/or back panel laminate (PL) is folded onto itself to form the barrier (5) and such that both the inner and outer nonwovens (NW1, NW2) are folded at one or more positions.

6. An absorbent article according to Claim 5 wherein at least the inner nonowoven (NW1) is a multi-layer nonwoven comprising at least two, preferably at least three, layers selected from a spunbond nonwoven and a meltblown nonwoven, more preferably said nonwoven being selected from a spunbond-meltblown nonwoven, a spunbond-meltblown-spunbond nonwoven, and a spunbond-meltblown-meltblown-spunbond nonwoven.

7. An absorbent article according to any of Claims 5 to 6 wherein the outer nonwoven (NW2) is a nonwoven that is the same or different to the inner nonwoven (NW1), preferably the outer nonwoven (NW2) comprising, more preferably consisting of, one or more spunbond nonwoven layers.

8. An absorbent article according to any of Claims 5 to 7 wherein said front and/or back panels (2, 3) comprise a tummy pause (TP) being free of said elastic strands or comprising elastic strands that have been rendered inelastic by deactivation, and wherein the barrier (5) overlaps said tummy pause (TP), preferably overlaps the entire width of said tummy pause (TP) along an axis parallel to the transversal centerline (x).

9. An absorbent article according to Claim 8 wherein the tummy pause (TP) has a width extending along an axis parallel to the transversal centerline (x), and said width being more than 60%, preferably more than 65%, more preferably from 70% to 180%, even more preferably from 75% to 150%, of a total width of the absorbent insert (4).

10. An absorbent article according to any of Claims 5 to 9 wherein the front and/or back panels (2, 3) comprise a first elastic region (ER1) and a second elastic region (ER2) wherein the first and second elastic regions (ER1, ER2) each comprise one or more elastic strands (11), and wherein the first and second elastic regions (ER1, ER2) are separated by a non-elastic region (NER), wherein the one or more positions of the fold(s) substantially correspond the non-elastic region (NER).

11. An absorbent article according to Claim 10 wherein the first elastic region (ER1) comprises a plurality of spaced-apart elastic strands (11) having one or more first strand spacings (ss1) and wherein the second elastic region (ER2) comprises one or more elastic strands (11).

12. An absorbent article according to any of Claims 10 to 11 wherein the second elastic region (ER2) comprises a plurality of spaced-apart elastic strands (11) having one or more second strand spacings (ss2) and wherein the first and second strand spacings are different, preferably wherein the first strand spacing(s) (ss1) is greater than the second strand spacing(s) (ss2), more preferably wherein the barrier (5) comprises said second elastic region (ER2).

13. An absorbent article according to any of Claims 10 to 12 wherein at least one of the elastic strands of the second elastic region (ER2) is coloured so that the barrier (5) is visually discernable from the rest of the article.

14. An absorbent article according to any of Claims 5 to 13 wherein the outer nonwoven (NW2) is joined to the inner nonwoven (NW1) at a first fold position (FP1), and wherein the inner nonwoven (NW1) is joined to itself at a second fold position (FP2), preferably wherein the first fold position (FP1) and the second fold position (FP2) are different, more preferably wherein the first and second fold positions (FP1, FP2) do not overlap.

15. An absorbent article according to any of the preceding Claims wherein the front and back panels (2, 3) are refastenably joined together to define a pair of side seams such that the seams can be opened and re-closed, preferably wherein the seams are maintained in closed state by one or more cooperating male and female fasteners preferably hook and loop fasteners respectively.

16. An absorbent article according to any of the preceding Claims wherein the absorbent core (8) comprises one or more channels (13) and at least one recessed region (R) positioned proximal and/or adjacent to the barrier (5), and wherein the recessed region has a width being at least 150% greater, preferably at least 200% greater, even more preferably from 2.5 to 9 times greater, than a width of the channel(s) (13); and/or wherein the absorbent core (8) comprises one or more channels (13) and at least one recessed region (R) positioned proximal and/or adjacent to the barrier (5), and wherein the channel(s) (13) and the at least one recessed region (R) are mutually separated by absorbent material.

17. An absorbent article according to any of the preceding Claims wherein the absorbent core (8) comprises one or more channels (13), preferably substantially free of absorbent material; each said channel being circumscribed by absorbent material; and preferably wherein the absorbent core (8) comprises a core wrap to enclose the absorbent material, and the channel(s) preferably being formed by joining top and bottom layers of said core wrap together.

18. An absorbent article according to any of the preceding Claims wherein the absorbent core (8) comprises a recessed region (R) proximal and/or adjacent to the barrier (5).

19. An absorbent article according to Claims 17 and 18 wherein the recessed region (R) and channel(s) (13) are mutually separated by absorbent material preferably such that the channel(s) are not directly connected to the recessed region but are rather spaced apart therefrom, preferably wherein the distance between the channel(s) and the recessed region is greater or equal to the width of the channel(s).

**Patentansprüche**

1. Absorbierender Artikel (1) für Körperhygiene, vorzugsweise eine Einwegunterhose, umfassend:

   - ein im Wesentlichen sich quer erstreckendes vorderes Feld (2);
   - ein im Wesentlichen sich quer erstreckendes hinteres Feld (3); und
   - eine sich im Wesentlichen in Längsrichtung erstreckende absorbierende Einlage(4), die mit jedem des vorderen und des hinteren Felds (2, 3) gefügt ist, die absorbierende Einlage (4) umfassend vordere und hintere Querkanten sowie linke und rechte Längskanten, die die vorderen und hinteren Querkanten verbinden, um einen Umfang davon auszubilden; und wobei die Einlage (4) einen absorbierenden Kern (8) umfasst;

   wobei das vordere und das hintere Feld (2, 3) zusammengefügt sind, um ein Paar von Seitennähten auszubilden, und **dadurch gekennzeichnet, dass** der Artikel eine quer verlaufende Taillenbarriere (5) proximal zu den vorderen und/oder hinteren Querkanten der absorbierenden Einlage (4) umfasst, wobei die Barriere (5) eine Vielzahl von flüssigkeitsbremsenden Schichten umfasst, wobei die Vielzahl von flüssigkeitsbremsenden Schichten mindestens zwei Schichten, die aus der Gruppe ausgewählt sind, bestehend aus hydrophoben Vliesstoffen und im Wesentlichen flüssigkeitsundurchlässigen Folien, und **dass** mindestens ein Großteil der Taillenbarriere (5) mindestens zwei der Vielzahl von flüssigkeitsbremsenden Schichten umfasst, und wobei die mindestens zwei der Vielzahl von flüssigkeitsbremsenden Schichten an einer oder mehreren Stellen nicht gefügt sind, sodass die Schichten an der/den Stelle(n) durch einen Spalt (G) voneinander getrennt sind, und vorzugsweise wobei die eine oder die mehreren Stellen zusammen einem Großteil einer Gesamtoberfläche der Taillenbarriere (5) entsprechen.

2. Absorbierender Artikel nach Anspruch 1, wobei die Taillenbarriere (5) mindestens zwei hydrophobe Vliesstoffe, die durch Klebstoff mit Unterbrechungen zusammengefügt sind, umfasst, vorzugsweise durch einen oder mehrere elastische Stränge, die mit Klebstoff strangbeschichtet und dazwischen angeordnet sind.

3. Absorbierender Artikel nach einem der vorstehenden Ansprüche, wobei der Artikel einen Sickerindex von weniger als 35 %, vorzugsweise weniger als 30 %, vorzugsweise weniger als 25 %, mehr bevorzugt weniger als 20 %, noch mehr bevorzugt weniger als 10 %, noch mehr bevorzugt von 0 % bis 5 %, gemäß dem Sickerindexverfahren hierin, aufweist.

4. Absorbierender Artikel nach einem der vorstehenden Ansprüche, wobei die hydrophoben Vliesstoffe synthetische Fasern umfassen, vorzugsweise daraus bestehen; vorzugsweise wobei die Fasern ein Material, ausgewählt aus Polypropylen oder Derivaten davon, Polyethylen oder Derivaten davon, Polymilchsäure oder Derivaten davon, Polymilch-co-Glycolsäure oder Derivaten davon, Polyhydroxyalkanoaten oder Derivaten davon und Gemischen davon, umfassen.

5. Absorbierender Artikel nach einem der vorstehenden Ansprüche, wobei das vordere und/oder das hintere Feld (2, 3) eine Vielzahl von elastischen Strängen (11), die zwischen zwei oder mehr hydrophoben Vliesstoffschichten angeordnet sind, umfassen, die Vliesstoffschichten umfassend mindestens einen inneren Vliesstoff (NW1) und einen äußeres Vliesstoff (NW2), die im Wesentlichen kongruent zueinander sind, um ein Laminat (PL) für das vordere/und oder das hintere Feld auszubilden; und wobei das Laminat (PL) für das vordere/und oder das hintere Feld auf sich selbst gefaltet ist, um die Barriere (5) auszubilden, und derart, dass sowohl der innere als auch der äußere Vliesstoff (NW1, NW2) an einer oder mehreren Positionen gefaltet sind.

6. Absorbierender Artikel nach Anspruch 5, wobei mindestens der innere Vliesstoff (NW1) ein mehrschichtiger Vliesstoff ist, umfassend mindestens zwei, vorzugsweise mindestens drei Schichten, die aus einem Spinnvliesstoff und einem Schmelzblasvliesstoff ausgewählt sind, wobei der Vliesstoff mehr bevorzugt aus einem spinngebundenen-schmelzgeblasenen Vliesstoff, einem spinngebundenen-schmelzgeblasenen-spinngebundenen Vliesstoff und einem spinngebundenen-schmelzgeblasenen-schmelzgeblasenen-spinngebundenen Vliesstoff ausgewählt ist.

7. Absorbierender Artikel nach einem der Ansprüche 5 bis 6, wobei der äußere Vliesstoff (NW2) ein Vliesstoff ist, der gleich oder verschieden von dem inneren Vliesstoff (NW1) ist, der äußere Vliesstoff (NW2) vorzugsweise umfassend, mehr bevorzugt bestehend aus, eine(r) oder mehreren Spinnvliesstoffschichten.

8. Absorbierender Artikel nach einem der Ansprüche 5 bis 7, wobei das vordere und/oder das hintere Feld (2, 3) eine Bauchaussparung (TP) umfassen, die frei von den elastischen Strängen ist oder umfassend elastische Stränge, die durch Deaktivierung unelastisch gemacht wurden, und wobei die Barriere (5) die Bauchaussparung (TP) überlappt, vorzugsweise die gesamte Breite der Bauchaussparung (TP) entlang einer Achse, parallel zu der Quermittellinie (x) überlappt.

9. Absorbierender Artikel nach Anspruch 8, wobei die Bauchaussparung (TP) eine Breite, die sich entlang einer Achse parallel zu der Quermittellinie (x) erstreckt, aufweist, und wobei die Breite mehr als 60 %, vorzugsweise mehr als 65 %, mehr bevorzugt 70 % bis 180 %, noch mehr bevorzugt 75 % bis 150 %, einer Gesamtbreite der absorbierenden Einlage (4) beträgt.

10. Absorbierender Artikel nach einem der Ansprüche 5 bis 9, wobei das vordere und/oder das hintere Feld (2, 3) einen ersten elastischen Bereich (ER1) und einen zweiten elastischen Bereich (ER2) umfassen, wobei der erste und der zweite elastische Bereich (ER1, ER2) jeder einen oder mehrere elastische Stränge (11) umfassen, und wobei der erste und der zweite elastische Bereich (ER1, ER2) durch einen nicht elastischen Bereich (NER) getrennt sind, wobei die eine oder die mehreren Positionen der Falte(n) im Wesentlichen dem nicht elastischen Bereich (NER) entsprechen.

11. Absorbierender Artikel nach Anspruch 10, wobei der erste elastische Bereich (ER1) eine Vielzahl von beabstandeten elastischen Strängen (11), die einen oder mehrere erste Strangabstände (ss1) aufweisen, umfasst, und wobei der zweite elastische Bereich (ER2) einen oder mehrere elastische Stränge (11) umfasst.

12. Absorbierender Artikel gemäß einem der Ansprüche 10 bis 11, wobei der zweite elastische Bereich (ER2) eine Vielzahl von beabstandeten elastischen Strängen (11), die einen oder mehrere zweite Strangabstände (ss2) aufweisen, umfasst, und wobei die ersten und die zweiten Strangabstände verschieden sind, vorzugsweise wobei der/die erste(n) Strangabstand/Strangabstände (ss1) größer als der/die zweite(n) Strangabstand/Strangabstände (ss2) ist/sind, mehr bevorzugt wobei die Barriere (5) den zweiten elastischen Bereich (ER2) umfasst.

13. Absorbierender Artikel nach einem der Ansprüche 10 bis 12, wobei mindestens einer der elastischen Stränge des zweiten elastischen Bereichs (ER2) gefärbt ist, sodass die Barriere (5) von dem Rest des Artikels sichtbar unterscheidbar ist.

14. Absorbierender Artikel nach einem der Ansprüche 5 bis 13, wobei der äußere Vliesstoff (NW2) mit dem inneren Vliesstoff (NW1) an einer ersten Faltposition (FP1) gefügt ist, und wobei der innere Vliesstoff (NW1) mit sich selbst an einer zweiten Faltposition (FP2) gefügt ist, vorzugsweise wobei die erste Faltposition (FP1) und die zweite Faltposition (FP2) verschieden sind, mehr bevorzugt wobei sich die erste und die zweite Faltposition (FP1, FP2) nicht überlappen.

15. Absorbierender Artikel nach einem der vorstehenden Ansprüche, wobei das vordere und das hintere Feld (2, 3) wiederbefestigbar zusammengefügt sind, um ein Paar von Seitennähten zu definieren, derart, dass die Nähte geöffnet und wieder geschlossen werden können, vorzugsweise wobei die Nähte durch einen oder mehrere zusammenwirkende männliche und weibliche Verschlüsse, vorzugsweise Klettverschlüsse, in geschlossenem Zustand gehalten werden.

16. Absorbierender Artikel nach einem der vorstehenden Ansprüche, wobei der absorbierende Kern (8) einen oder mehrere Kanäle (13) und mindestens einen vertieften Bereich (R), die proximal und/oder angrenzend zu der Barriere (5) positioniert sind, umfasst, und wobei der vertiefte Bereich eine Breite, die mindestens 150 % größer, vorzugsweise mindestens 200 % größer, noch mehr bevorzugt von 2,5- bis 9-mal größer, als eine Breite des Kanals der /Kanäle (13) ist, aufweist; und/oder wobei der absorbierende Kern (8) einen oder mehrere Kanäle (13) und mindestens einen vertieften Bereich (R), die proximal und/oder angrenzend zu der Barriere (5) positioniert sind, umfasst, und wobei der Kanal/die Kanäle (13) und der mindestens eine vertiefte Bereich (R) durch absorbierendes Material voneinander getrennt sind.

17. Absorbierender Artikel nach einem der vorstehenden Ansprüche, wobei der absorbierende Kern (8) einen oder mehrere Kanäle (13), die vorzugsweise im Wesentlichen frei von absorbierendem Material sind, umfasst; wobei jeder Kanal von absorbierendem Material umgeben ist; und vorzugsweise wobei der absorbierende Kern (8) eine Kernumhüllung, um das absorbierende Material zu umschließen, umfasst, und der Kanal/die Kanäle vorzugsweise durch das Zusammenfügen von oberen und unteren Schichten der Kernumhüllung ausgebildet werden.

18. Absorbierender Artikel nach einem der vorstehenden Ansprüche, wobei der absorbierende Kern (8) einen vertieften Bereich (R) proximal und/oder angrenzend zu der Barriere (5) umfasst.

19. Absorbierender Artikel nach den Ansprüchen 17 und 18, wobei der vertiefte Bereich (R) und der Kanal /die Kanäle (13) durch absorbierendes Material voneinander getrennt sind, vorzugsweise derart, dass der Kanal/die Kanäle nicht mit dem vertieften Bereich direkt verbunden sind, sondern eher von diesem beabstandet sind, vorzugsweise wobei der Abstand zwischen dem Kanal/den Kanälen und dem vertieften Bereich größer oder gleich der Breite des Kanals/der Kanäle ist.

## Revendications

1. Article absorbant (1) destiné à l'hygiène personnelle, de préférence une culotte jetable, comprenant :

   - un panneau avant (2) s'étendant sensiblement transversalement ;
   - un panneau arrière (3) s'étendant sensiblement transversalement ; et
   - un insert absorbant (4) s'étendant sensiblement longitudinalement, relié à chacun desdits panneaux avant et arrière (2, 3), ledit insert absorbant (4) comprenant des bords transversaux avant et arrière et des bords longitudinaux gauche et droit reliant les bords transversaux avant et arrière pour former un périmètre de celui-ci ; et dans lequel ledit insert (4) comprend une âme absorbante (8) ;

   dans lequel les panneaux avant et arrière (2, 3) sont reliés ensemble pour définir une paire de coutures latérales, et **caractérisé en ce que** ledit article comprend une barrière transversale à la taille (5) proximale aux bords transversaux avant et/ou arrière de l'insert absorbant (4) dans lequel ladite barrière (5) comprend une pluralité de couches retardatrices de liquide dans lequel ladite pluralité de couches retardatrices de liquide comprend au moins deux couches choisies parmi le groupe constitué par des non-tissés hydrophobes, et des films sensiblement imperméables au liquide, et **en ce qu'**au moins une majorité de la barrière à la taille (5) comprend au moins deux de ladite pluralité de couches retardatrices de liquide et dans lequel lesdites au moins deux de ladite pluralité de couches retardatrices de liquide ne sont pas reliées au niveau d'un ou plusieurs endroits de sorte que lesdites couches sont séparées les unes des autres au niveau dudit ou desdits endroits par un espace (G), et de préférence dans lequel lesdits un ou plusieurs endroits correspondent ensemble à une majorité d'une surface totale de ladite barrière à la taille (5).

2. Article absorbant selon la revendication 1, dans lequel la barrière à la taille (5) comprend au moins deux non-tissés hydrophobes reliés ensemble de manière intermittente par un adhésif, de préférence par un ou plusieurs fils élastiques qui sont revêtus d'un adhésif et pris en sandwich entre eux.

3. Article absorbant selon l'une quelconque des revendications précédentes, dans lequel l'article a un indice de suintement inférieur à 35 %, de préférence inférieur à 30 %, de préférence inférieur à 25 %, plus préférablement inférieur à 20 %, encore plus préférablement inférieur à 10 %, encore plus préférablement entre 0 % et 5 %, selon la méthode de l'indice de suintement décrite ici.

4. Article absorbant selon l'une quelconque des revendications précédentes, dans lequel les non-tissés hydrophobes comprennent des fibres synthétiques, de préférence en sont constitués ; de préférence dans lequel lesdites fibres comprennent un matériau choisi parmi le polypropylène ou les dérivés de celui-ci, le polyéthylène ou les dérivés de celui-ci, l'acide polylactique ou les dérivés de celui-ci, l'acide polylactique-co-glycolique ou les dérivés de celui-ci, les polyhydroxyalcanoates ou les dérivés de ceux-ci, et des mélanges de ceux-ci.

5. Article absorbant selon l'une quelconque des revendications précédentes, dans lequel les panneaux avant et/ou arrière (2, 3) comprennent une pluralité de fils élastiques (11) pris en sandwich entre deux couches de non-tissé hydrophobe ou plus, lesdites couches de non-tissé comprenant au moins un non-tissé intérieur (NW1) et un non-tissé extérieur (NW2) qui sont sensiblement congruents entre eux pour former un panneau stratifié avant et/ou arrière (PL) ; et dans lequel le panneau stratifié avant et/ou arrière (PL) est plié sur lui-même pour former la barrière (5) et de telle sorte que les non-tissés intérieur et extérieur (NW1, NW2) sont pliés au niveau d'un ou plusieurs positions.

6. Article absorbant selon la revendication 5, dans lequel au moins le non-tissé intérieur (NW1) est un non-tissé multicouche comprenant au moins deux, de préférence au moins trois, couches choisies parmi un non-tissé filé-lié et un non-tissé de fusion-soufflage, plus préférablement ledit non-tissé étant choisi parmi un non-tissé filé-lié/de fusion-soufflage, un non-tissé filé-lié/de fusion-soufflage/filé-lié, et un non-tissé filé-lié/de fusion-soufflage/de fusion-soufflage/filé-lié.

7. Article absorbant selon l'une quelconque des revendications 5 à 6, dans lequel le non-tissé extérieur (NW2) est un non-tissé qui est identique ou différent du non-tissé intérieur (NW1), de préférence le non-tissé extérieur (NW2) comprenant, plus préférablement constitué de, une ou plusieurs couches de non-tissé filé-lié.

8. Article absorbant selon l'une quelconque des revendications 5 à 7, dans lequel lesdits panneaux avant et/ou arrière (2, 3) comprennent une pause ventrale (TP) étant dépourvue de fils élastiques ou comprenant des fils élastiques qui ont été rendus inélastiques par désactivation, et dans lequel la barrière (5) recouvre ladite pause ventrale (TP), de préférence recouvre toute la largeur de ladite pause ventrale (TP) le long d'un axe parallèle à la ligne centrale transversale (x).

9. Article absorbant selon la revendication 8, dans lequel la pause ventrale (TP) a une largeur s'étendant le long d'un axe parallèle à la ligne centrale transversale (x), et ladite largeur étant supérieure à 60 %, de préférence supérieure à 65 %, plus préférablement de 70 % à 180 %, encore plus préférablement de 75 % à 150 %, d'une largeur totale de l'insert absorbant (4).

10. Article absorbant selon l'une quelconque des revendications 5 à 9, dans lequel les panneaux avant et/ou arrière (2, 3) comprennent une première région élastique (ER1) et une seconde région élastique (ER2), dans lequel la première et la seconde région élastique (ER1, ER2) comprennent chacune un ou plusieurs fils élastiques (11), et dans lequel la première et la seconde région élastique (ER1, ER2) sont séparées par une région non élastique (NER), dans lequel la ou les positions du ou des plis correspondent sensiblement à la région non élastique (NER).

11. Article absorbant selon la revendication 10, dans lequel la première région élastique (ER1) comprend une pluralité de fils élastiques espacés (11) ayant un ou plusieurs premiers espacements de fils (ss1) et dans lequel la seconde région élastique (ER2) comprend un ou plusieurs fils élastiques (11).

12. Article absorbant selon l'une quelconque des revendications 10 à 11, dans lequel la seconde région élastique (ER2) comprend une pluralité de fils élastiques espacés (11) ayant un ou plusieurs seconds espacements de fils (ss2) et dans lequel les premiers et seconds espacements de fils sont différents, de préférence dans lequel le ou les premiers espacements de fils (ss1) sont supérieurs au ou aux seconds espacements de fils (ss2), plus préférablement dans lequel la barrière (5) comprend ladite seconde région élastique (ER2).

13. Article absorbant selon l'une quelconque des revendications 10 à 12, dans lequel au moins l'un des fils élastiques de la seconde région élastique (ER2) est coloré de sorte que la barrière (5) est visuellement discernable du reste de l'article.

14. Article absorbant selon l'une quelconque des revendications 5 à 13, dans lequel le non-tissé extérieur (NW2) est relié au non-tissé intérieur (NW1) au niveau d'une première position de pliage (FP1), et dans lequel le non-tissé intérieur (NW1) est relié à lui-même au niveau d'une seconde position de pliage (FP2), de préférence dans lequel la première position de pliage (FP1) et la seconde position de pliage (FP2) sont différentes, plus préférablement dans lequel la première et la seconde position de pliage (FP1, FP2) ne se chevauchent pas.

15. Article absorbant selon l'une quelconque des revendications précédentes, dans lequel les panneaux avant et arrière (2, 3) sont reliés ensemble de manière réattachable pour définir une paire de coutures latérales de telle sorte que les coutures peuvent être ouvertes et refermées, de préférence dans lequel les coutures sont maintenues à l'état fermé par une ou plusieurs fermetures mâles et femelles coopérantes, de préférence des fermetures autoaggripantes respectivement.

16. Article absorbant selon l'une quelconque des revendications précédentes, dans lequel l'âme absorbante (8) comprend un ou plusieurs canaux (13) et au moins une région en retrait (R) positionnée de manière proximale et/ou adjacente à la barrière (5), et dans lequel la région en retrait a une largeur supérieure d'au moins 150 %, de préférence d'au moins 200 %, encore plus préférablement supérieure de 2,5 à 9 fois, à une largeur du ou des canaux (13) ; et/ou dans lequel l'âme absorbante (8) comprend un ou plusieurs canaux (13) et au moins une région en retrait (R) positionnée de manière proximale et/ou adjacente à la barrière (5), et dans lequel le ou les canaux (13) et l'au moins une région en retrait (R) sont mutuellement séparés par un matériau absorbant.

17. Article absorbant selon l'une quelconque des revendications précédentes, dans lequel l'âme absorbante (8) comprend un ou plusieurs canaux (13), de préférence sensiblement dépourvus de matériau absorbant ; chacun desdits canaux étant entouré d'un matériau absorbant ; et de préférence dans lequel l'âme absorbante (8) comprend une enveloppe d'âme pour enfermer le matériau absorbant, et le ou les canaux étant de préférence formés en reliant les couches supérieure et inférieure de ladite enveloppe d'âme.

18. Article absorbant selon l'une quelconque des revendications précédentes, dans lequel l'âme absorbante (8) comprend une région en retrait (R) proximale et/ou adjacente à la barrière (5).

19. Article absorbant selon les revendications 17 et 18, dans lequel la région en retrait (R) et le ou les canaux (13) sont mutuellement séparés par un matériau absorbant, de préférence de telle sorte que le ou les canaux ne soient pas directement reliés à la région en retrait mais soient plutôt espacés de celle-ci, de préférence dans lequel la distance entre le ou les canaux et la région en retrait est supérieure ou égale à la largeur du ou des canaux.

FIG. 1

FIG. 2

FIG. 3

FIG. 4

**FIG. 5**

**FIG. 6**

**FIG. 7**

**FIG. 8**

**FIG. 9**

FIG. 10

FIG. 11A

FIG. 11B

FIG. 12

FIG. 13

EP 4 458 337 B1

FIG. 14

FIG. 15

FIG. 17

**FIG.18A**

**FIG.18B**

**FIG.18C**

**FIG.18D**

FIG. 19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5151092 A, Buell
- WO 2008155699 A
- WO 2012052172 A
- EP 3451989 B1
- WO 2021170027 A1
- EP 3453368 A
- EP 3342386 A
- WO 2012170778 A
- WO 2012170779 A
- WO 2012170781 A
- WO 20121708008 A
- WO 9516746 A
- US 5938648 A
- US 4681793 A
- US 5865823 A
- US 5571096 A
- US 6946585 B2